# EUROPEAN PATENT SPECIFICATION

(11) **EP 3 259 345 B1**
(45) Date of publication and mention of the grant of the patent: **08.02.2023**
(21) Application number: 16705202.6
(22) Date of filing: 19.02.2016
(51) Int. Cl.: C12N 5/071

(54) **USE OF A LAMININ FOR DIFFERENTIATING PLURIPOTENT CELLS INTO HEPATOCYTE LINEAGE CELLS**
VERWENDUNG EINES LAMININS ZUR DIFFERENZIERUNG VON PLURIPOTENTEN ZELLEN IN ZELLEN DER HEPATOCYTEN-LINIE
UTILISATION D'UNE LAMININE POUR LA DIFFÉRENCIATION DE CELLULES PLURIPOTENTES EN CELLULES DE LIGNÉE D'HÉPATOCYTES

(30) Priority: 20.02.2015 EP 15305265
(43) Date of publication of application: 27.12.2017
(73) Proprietor: INSERM (Institut National de la Santé et de la Recherche Médicale), 75654 Paris Cedex 13 (FR); Nantes Université, 44000 Nantes (FR)
(72) Inventor: NGUYEN, Tuan Huy, 44300 Nantes (FR); FOURRIER, Angélique, 44300 Nantes (FR)
(74) Representative: Ernest Gutmann - Yves Plasseraud S.A.S.
(86) International application number: PCT/EP2016/053560
(87) International publication number: WO 2016/131959

(56) References cited:
- US-A1- 2005 042 748
- US-A1- 2007 254 359
- KAZUO TAKAYAMA ET AL: "Long-Term Self-Renewal of Human ES/iPS-Derived Hepatoblast-like Cells on Human Laminin 111-Coated Dishes", STEM CELL REPORTS, vol. 1, no. 4, 1 October 2013 (2013-10-01) , pages 322-335, XP055190506, ISSN: 2213-6711, DOI: 10.1016/j.stemcr.2013.08.006
- MITSURU INAMURA1 ET AL: "Efficient Generation of Hepatoblasts From Human ES Cells and iPS Cells by Transient verexpression of Homeobox Gene HEX", MOLECULAR THERAPY, , vol. 19, no. 2 23 November 2010 (2010-11-23), pages 400-407, XP008155514, DOI: 10.1038/MT.2010.241 Retrieved from the Internet: URL:http://www.nature.com/mt/journal/vaop/ ncurrent/full/mt2010241a.html [retrieved on 2010-11-23]
- KARIM SI-TAYEB ET AL: "Highly efficient generation of human hepatocyte-like cells from induced pluripotent stem cells", HEPATOLOGY, JOHN WILEY & SONS, INC, USA, vol. 51, no. 1, 1 January 2010 (2010-01-01), pages 297-305, XP002667016, ISSN: 0270-9139, DOI: 10.1002/HEP.23354 [retrieved on 2009-10-01]
- R.E. SCHWARTZ ET AL: "Pluripotent stem cell-derived hepatocyte-like cells", BIOTECHNOLOGY ADVANCES, vol. 32, no. 2, 1 March 2014 (2014-03-01), pages 504-513, XP055190576, ISSN: 0734-9750, DOI: 10.1016/j.biotechadv.2014.01.003
- YAMATO KIKKAWA ET AL: "Maintenance of hepatic differentiation by hepatocyte attachment peptides derived from laminin chains", JOURNAL OF BIOMEDICAL MATERIALS RESEARCH PART A, vol. 99A, no. 2, 16 November 2011 (2011-11-16), pages 203-210, XP055190691, ISSN: 1549-3296, DOI: 10.1002/jbm.a.33176
- A. DELAFOREST ET AL: "HNF4A is essential for specification of hepatic progenitors from human pluripotent stem cells", DEVELOPMENT, vol. 138, no. 19, 1 October 2011 (2011-10-01), pages 4143-4153, XP055194344, ISSN: 0950-1991, DOI: 10.1242/dev.062547
- KATE CAMERON ET AL: "Recombinant Laminins Drive the Differentiation and Self-Organization of hESC-Derived Hepatocytes", STEM CELL REPORTS, vol. 5, no. 6, 1 December 2015 (2015-12-01), pages 1250-1262, XP055259071, United States ISSN: 2213-6711, DOI: 10.1016/j.stemcr.2015.10.016
- Takumi Teratani ET AL: "Direct hepatic fate specification from mouse embryonic stem cells", Hepatology, vol. 41, no. 4, 1 April 2005 (2005-04-01), pages 836-846, XP055624912, ISSN: 0270-9139, DOI: 10.1002/hep.20629

## Description

### FIELD OF THE INVENTION

The invention relates to the field of cell differentiation and cell therapy.

### BACKGROUND OF THE INVENTION

Since their discovery, human induced pluripotent cells (hiPSC) and human embryonic stem cells (hESC) has been intensively investigated as a renewable source of any specialized cells of the body for replacing diseased cells [1, 2]. They have been envisioned for the treatment of a wild range of diseases, such as severe cardiac [3], neurological [4], liver [5] and retinal disease [6], and diabetes [7, 8].

Liver disease affects millions of people worldwide. To date, liver transplantation is the only curative option when patients with severe metabolic liver disorders. About 37,000 patients are on the waiting lists for liver transplantation in Europe and United States. More than 5500 liver transplantations are performed in Europe per year and inherited metabolic diseases represent 26% of the indications (European Liver Transplant Registry). However, less than one third of patients on the liver waiting list can receive a liver transplant each year and the number of patients dying while on the waiting lists for liver transplantation (about 10%) has increased during the last years as a result of the shortage of donor organs.

Over the past 15 years, transplantation of hepatocytes isolated from cadaveric livers into the patients' liver has emerged as a clinical alternative to liver transplantation [9, 10], particularly for inborn metabolic diseases where liver functions are maintained and there is a single enzymatic defect. Unequivocal evidence of the metabolic function of transplanted human hepatocytes was first demonstrated in a patient with type 1 Crigler-Najjar (CN1) [11]. After cell transplantation, patient's clinical status and life quality were improved with restoration of 5% UGT1A1 activity leading to 50% reduction in bilirubinemia and in phototherapy treatment. This seminal study opened the way for treating more than 40 patients affected with inherited metabolic liver disease, mostly with CN1 and urea cycle disorders, but also type 1 glycogen storage disease, infantile Refsum's disease, progressive type 2 familial intrahepatic cholestasis, and type VII hemophilia [9]. Hepatocyte transplantation permitted some children to avoid new neurological damages while waiting for liver transplantation. A relatively small amount of infused hepatocytes (1.5-2×10⁹ cells) was sufficient to improve the metabolic deficiency of some affected patients. However, long-lasting stabilization effect certainly requires repeated cell infusions [12]. This approach suffers other important limitations for its general and routine use in clinical practice. Available liver transplants are prioritized for liver transplantation and only those of marginal quality were used for hepatocyte isolation. Thereby, isolated hepatocytes are of variable quality and quantity. They rapidly dedifferentiate and cannot be expanded in culture, and do not tolerate cryopreservation well. Collectively, these limitations emphasize the need to explore other sources of functional human hepatic cells.

These last years, several groups reported the in vitro differentiation of hESCs and hiPSCs into hepatocyte-like cells (HLCs) using diverse culture conditions mimicking liver embryonic development stages [5, 13-21]. HLCs did not display functions of fully mature hepatocyte, but had a phenotype closer to fetal human hepatocytes, expressing for instance AFP [22]. They can engraft, expand in some extend and maintain hepatic functionality after transplantation in the livers of immuno-compromised animals that were chemically-injured, irradiated or genetically-manipulated to give a selective growth advantage to transplanted hepatocytes, such as mice over-expressing the urokinase-type plasminogen activator for inducing a constitutive recipient liver injury and regenerative stimuli [5, 14, 17, 23-25, 60].

Some studies investigated the therapeutic potential of HLCs and successfully treated various murine models of chemically-induced lethal hepatic failure [26-33]. Nevertheless, the liver has a remarkable ability to regenerate itself from endogenous hepatocytes. Thereby, a temporary support of hepatic metabolic functions by HLCs is sufficient to rescue mice from acute liver failure. By contrast, for treatment of inherited liver diseases, HLCs must fully differentiate in vivo and be functional in long-term to express the missing mature hepatic metabolic functions in the diseased animals. So far, successful metabolic correction of an animal modeling a human inherited liver disease in which transplanted HLCs have no selective survival and growth advantage in the recipient livers has not been demonstrated yet.

The Gunn rat, an animal of CN1, has a natural mutation in UGT1A1 causing a complete loss of UGT1A1 activity and hyperbilirubinemia soon after birth. The total absence of bilirubin conjugates in the bile provides an easy, unequivocal and sensitive read out to evaluate the restoration of UGT1A1 activity. Thus, the Gunn rat constitutes an invaluable and convenient model to follow in vivo hepatic cell maturation and therapeutic potential of transplanted hepatocytes. As in CN1 patients, a partial restoration of UGT1A1 activity after hepatocyte transplantation results in a significant metabolic correction [37-39].

Before consideration of HLCs for clinical use, a crucial issue is also to produce them using hepatic differentiation protocol that is in GMP (Good Manufacturing Practices)-compatible. Current protocols of hepatic differentiation commonly contained feeder cells-conditioned medium, serum, animal-derived matrices (such as MatrigelTM), mouse feeder cells, viral vectors for improving hepatic differentiation, or small molecules that are not available in GMP [15, 34-36].

Previous works discloses such as in US2005/042748 and the corresponding publication [69] disclose a method for inducing hepatic differentiation of pluripotent cells into hepatocytes, wherein pluripotent cells are first differentiated on gelatin-coated culture dishes. Other authors disclose the differentiation of human pluripotent stem cells (hPSCs) into hepatoblast-like cells (HBCs) and their proliferation, wherein hPSCs are first differentiated on Matrigel-coated dishes [66], or disclose the differentiation of human embryonic stem cells into hepatocytes like cells using Matrigel [68] or. discloses the differentiation of human embryonic stem cells and induced pluripotent stem cells into hepatoblasts by overexpression of the homeobox gene HEX [67].

All of which are sources of unknown factors that render the resulting tissues incompatible with future clinical applications.

Accordingly, there is a clinical demand of human hepatocytes for allogeneic cell therapies. Pluripotent stem cells have been intensively investigated as a renewable source of transplantable HLCs. However, the demonstration that HLCs could treat an inherited metabolic liver disease in absence of any selective growth advantage of transplanted cells over resident rodent hepatocytes is still waiting. In addition, previously generated HLCs were produced using protocols that are rendering them unsuitable for clinical applications.

Recently, laminins such as laminin-521 (LN-521) and laminin-111 (LN-111) have been described as a relevant matrix for cell culture, more particularly for maintaining functional properties of long-term in vitro culture of cells of interest such as pluripotent cells or HLCs however laminins have never been disclosed nor suggested as useful for inducing and/or improving hepatic differentiation. In addition, no study has suggested that laminin matrices employed alone could support initiation and subsequent hepatocyte differentiation from pluripotent stem cell lines.

Thus, international patent application WO 2012/080844 relates to a new use of laminin-521. Indeed, laminin-521 can maintain stem cells in vitro pluripotency, enable self-renewal, and enable single cell survival of human embryonic stem cells. When pluripotent human embryonic stem cells are cultured on plates coated with recombinant laminin-521 in the absence of differentiation inhibitors or feeder cells, the embryonic stem cells proliferate and maintain their pluripotency.

Moreover, it has been shown that human hepatoblast-like cells can be first generated from pluripotent cells on Matrigel-coated dishes (induction of hepatic differentiation) and then cultured on laminin-111-coated dishes for expanding them for more than 3 months while keeping the potential ability to differentiate into both hepatocyte-like cells and cholangiocyte-like cells [36].

Surprisingly, the Applicant discovered a novel role of LN-111 and LN-521 in hepatocyte-like cells differentiation. This novel role for LN-521 is opposite to the role of pluripotency maintenance described in WO2012/080844.

### SUMMARY OF THE INVENTION

In a first aspect, the invention relates to a method for inducing human hepatic differentiation comprising the steps of culturing a population of human pluripotent or multipotent cells on a support coated with human laminin in an endoderm induction medium to produce a population of human definitive endoderm (DE) cells and wherein said method does not comprise the overexpression of homeobox transgene HEX in said population of cells.

In a second aspect, the invention relates to a method for inducing human hepatic differentiation comprising the steps of:
(i) culturing a population of human definitive endoderm (DE) cells on a support coated with human laminin in a hepatic induction medium to produce a population of human hepatoblast-like cells, and
(ii) optionally culturing said population of human hepatoblast-like cells on a support coated with human laminin in a hepatic maturation medium to produce a population of hepatocyte-like cells, preferably human fetal hepatocyte-like cells and
wherein said method does not comprise the overexpression of homeobox transgene *HEX* in said population of cells.

In a third aspect, the invention relates to a method for inducing human hepatic differentiation comprising the steps of culturing a population of human pluripotent or multipotent cells on a support coated with human laminin in an endoderm induction medium to produce a population of human definitive endoderm (DE) cells, culturing said population of human DE cells on a support coated with human laminin in a hepatic induction medium to produce a population of human hepatoblast-like cells, and optionally culturing said population of human hepatoblast-like cells on a support coated with human laminin in a hepatic maturation medium to produce a population of hepatocyte-like cells, preferably human fetal hepatocyte-like cells and wherein said method does not comprise the overexpression of homeobox transgene HEX in said population of cells

In a fourth aspect, the invention relates to a method for inducing hepatic differentiation as above mentioned wherein the hepatic induction medium is a chemically defined medium comprising bone morphogenetic protein 4 (BMP4) and family growth factor (FGF10 or FGF2).

In a fifth aspect, the invention relates to a method for inducing hepatic differentiation as above mentioned wherein the hepatic maturation medium is a chemically defined medium comprising hepatic growth factor (HGF) and oncostatin M (OSM).

In a sixth aspect, the invention relates to a method for inducing hepatic differentiation as above mentioned wherein the endoderm induction medium is a chemically defined medium comprising at least Activin A and optionally WNT3A.

In a seventh aspect, the invention relates to a method for inducing hepatic differentiation as above mentioned wherein the hepatic induction medium and/or the endoderm induction medium further comprises a ROCK inhibitor and/or CHIR99021.

In an eighth aspect, the invention relates to a method for inducing hepatic differentiation as above mentioned wherein the human laminin (LN) is selected from the group consisting of laminin-111 (LN-111), laminin-211 (LN-211), laminin-332 (LN-332), laminin-411 (LN-411), laminin-421 (LN-421), laminin-511 (LN-511) and laminin-521 (LN-521).

In the ninth aspect, the invention relates to a method for inducing hepatic differentiation as above mentioned wherein the human laminin (LN) is selected from the group consisting of human recombinant laminin-111 (LN-111) and human recombinant laminin-521 (LN-521) and a mixture thereof.

### DETAILED DESCRIPTION OF THE INVENTION

The invention addresses these needs, as it relates to the identification of a chemically defined medium useful for differentiating a population of pluripotent cells or a population of definitive endoderm (DE) cells into a population of hepatocyte lineage cells. The inventors have surprisingly showed that laminin-111, alone, is as efficient as matrigel for initiating and supporting hepatic differentiation of hiPSC in our culture conditions, but with a recombinant matrix.

The inventors have then shown that laminin-521 (LN-521) is also useful as a matrix and allowed better differentiation of hiPSC into definitive endoderm and HLCs.

The inventors indeed have produced hepatoblasts derived from human pluripotent stem cells (IDHBs) in xeno-free, feeder-free and chemically defined protocols using recombinant laminin-111 (LN-111) as an extracellular matrix for initiating and supporting hepatic differentiation process. These IDHBs with a particular set of markers (combination of expressed or non-expressed markers) were transplanted in the liver of the Gunn rat, an animal model for Crigler-Najjar syndrome, which is characterized by high levels of unconjugated bilirubin. Following cell transplantation, they showed significant correction of hyperbilirubinemia, which remained stable throughout the 6 months study with no adverse events. They also showed that the transplanted IDHBs underwent further maturation *in situ* to restore the deficient metabolic hepatic function (bilirubin glucuronidation by UGT1A1). In conclusion, they demonstrated for the first time the efficacy of hiPSC-based regenerative with a GMP-compatible protocol for treating an inherited hepatic metabolic disease in absence of any selective growth advantage of transplanted cells over resident hepatocytes, which is the situation encountered in hepatocyte transplantation in humans.

### Methods for hepatic differentiation and uses of laminins

Accordingly, in a first aspect the invention relates to the use of a laminin (LN) as a matrix for hepatic differentiation.

The invention also relates to the use of a LN for inducing and/or improving differentiation of a population of pluripotent cells, a population of multipotent cells, or a population of definitive endoderm (DE) cells into a population of hepatocyte lineage cells.

As used herein, the term "laminin" (LN) refers to a protein of a family of heterotrimeric glycoproteins that reside primarily in the basal lamina. They function via binding interactions with neighboring cell receptors on the one side, and by binding to other laminin molecules or other matrix proteins such as collagens, nidogens or proteoglycans. The laminin molecules are also important signaling molecules that can strongly influence cellular behavior and function. Laminins are important in both maintaining cell/tissue phenotype, as well as in promoting cell growth and differentiation in tissue repair and development. Laminins are large, multi-domain proteins, with a common structural organization. The laminin molecule integrates various matrix and cell interactive functions into one molecule. A laminin protein molecule comprises one a-chain subunit, one β-chain subunit, and one γ-chain subunit, all joined together in a trimer through a coiled-coil domain. The twelve known laminin subunit chains can form at least 15 trimeric laminin types in native tissues. Within the trimeric laminin structures are identifiable domains that possess binding activity towards other laminin and basal lamina molecules, and membrane-bound receptors.

It should be further noted that the term "laminin" encompasses laminin either intact, as separate chains, or as fragments thereof. As used herein, the term "intact" refers to the protein being composed of all of the domains of the α-chain, β-chain, and γ-chain, with the three chains being joined together to form the heterotrimeric structure. The protein is not broken down into separate chains, fragments, or functional domains. For instance, laminin-111 and laminin-521 (as described below) are intact proteins. As used herein, the term "chain" refers to the entirety of the alpha, beta, or gamma chain of the laminin protein. As used herein, the term "fragment" refers to any protein fragment which contains one, two, or three functional domains that possesses binding activity to another molecule or receptor. However, a chain should not be considered a fragment because each chain possesses more than three such domains. Similarly, an intact laminin protein should not be considered a fragment. Examples of functional domains include Domains I, II, III, IV, V, VI, and the G domain.

There exist five different alpha chains, three beta chains and three gamma chains that in human tissues have been found in at least fifteen different combinations. These molecules are termed laminin-1 to laminin-15 based on their historical discovery, but an alternative nomenclature describes the isoforms based on their chain composition, e.g. laminin-111 (laminin-1) that contains alpha-1, beta-1 and gamma-1 chains. Four structurally defined family groups of laminins have been identified. The first group of five identified laminin molecules all share the β1 and γ1 chains, and vary by their a-chain composition (α1 to α5 chain). The second group of five identified laminin molecules, including laminin-521, all share the β2 and γ1 chain, and again vary by their a-chain composition. The third group of identified laminin molecules has one identified member, laminin-332, with a chain composition of α3β3γ2. The fourth group of identified laminin molecules has one identified member, laminin-213, with the newly identified γ3 chain (α2β1γ3).

At least 15 subtypes (or "isoforms") of laminin (LN) have been identified, based on their chain composition, including LN-111 (α1β1γ1), LN-121 (α1β2γ1), LN-211 (α2β1γ1), LN-213 (α2β1γ3), LN-221 (α2β2γ1), LN-311 (α3β3γ1), LN-321 (α3β2γ1), LN-332 (α3β3γ2), LN-411 (α4β1γ1), LN-421 (α4β2γ1), LN-423 (α4β2γ3), LN-511 (α5β1γ1), LN-521 (α5β2γ1), LN-522 (α5β2γ2), and LN-523 (α5β2γ3).

In one embodiment of the invention, said laminin is selected from the group consisting of laminin-111 (LN-111), laminin-211 (LN-211), laminin-332 (LN-332), laminin-411 (LN-411), laminin-421 (LN-421), laminin-511 (LN-511) and laminin-521 (LN-521).

In one embodiment of the invention, said laminin is a human laminin.

In one embodiment of the invention, said laminin is a human recombinant laminin.

In a preferred embodiment of the invention, said laminin is recombinant human laminin-111 (LN-111) and/or recombinant human laminin-521 (LN-521).

Laminin 521 (composed of α5, β2, γ1 chains) is expressed during early embryonic development, is secreted by human pluripotent stem cells and is known to stimulate their robust proliferation [63]. Laminin α5 is present in bile ducts and hepatic blood vessels (hepatic arteries, portal veins), except in sinusoids and thus laminin α5 is not normally associated with normal hepatocytes. In addition, the laminin β2 chain, and thus laminin-521, is not expressed in adult and normal animal liver [64].

The laminin α1 chain, and thus Laminin-111 (composed of α1, β1, γ1 chains), is not expressed in adult animal liver [65].

Thus, laminins extracted from adult liver tissues do not contain laminin 521 or Laminin-111.

As used herein, the term "recombinant" polypeptide refers to a polypeptide which is produced by expression from an encoding nucleic acid molecule. Systems for cloning and expression of a polypeptide in a variety of different host cells are well known.

When expressed in recombinant form, the polypeptide is preferably generated by expression from an encoding nucleic acid in a host cell. Any host cell may be used, depending upon the individual requirements of a particular system. Suitable host cells include bacteria mammalian cells, plant cells, yeast and baculovirus systems.

Recombinant human laminins such as recombinant human LN-111 or LN-521 can be purchased from Biolamina, Sundbyberg, Sweden.

In one embodiment of the invention, laminin is coated to the support such as a plate from 0.5 to 50 micrograms per milliliter (µg/mL), preferably from 1 to 10 µg/mL, more preferably at 5 µg/mL.

As used herein, the term "matrix" refers to a component/material (natural, synthetic or a combination thereof) forming a polymeric network which provides to *in vitro* cultured cells (e.g. on culture vessel such as flat plasticware) an *in vivo* like morphology and physiologically relevant environments for more realistic cell biology and better intercellular interactions for cell culture, facilitating cell attachment, growth, differentiation

In one embodiment, the matrix comprises a laminin such as recombinant human laminin-111 (LN-111) or recombinant human laminin-521 (LN-521) or a mixture thereof.

In another embodiment, the matrix comprises LN-521 and LN-111 in a ratio of about 5%/95%; 10%/90%; 20%/80%; 25%/75%; 30%/70%; 40%/60%; 50%/50%, 60%/40%; 70%/30%; 75%/25%, 80%/20%; 90%/10%; 95%/5%.

In another embodiment, the matrix comprises a mixture of a laminin and another component (such as matrix proteins including collagen I and fibronectin).

As used herein, the term "population" refers to a population of cells, wherein the majority (e.g., at least about 50%, preferably at least about 60%, more preferably at least about 70%, and even more preferably at least about 80%) of the total number of cells have the specified characteristics of the cells of interest for at least one of the markers of interest (e.g. a population of human hepatocyte-like cells comprises at least about 60%, preferably at least about 70%, more preferably at least about 80% of cells which have the hepatic functions and which express the markers typically expressed by human hepatocyte-like cells listed below such as for instance hepatocyte nuclear factor 4alpha (HNF4α)).

As used herein, the term "marker" refers to a protein, glycoprotein, or other molecule expressed on the surface of a cell or into a cell, and which can be used to help identify the cell. A marker can generally be detected by conventional methods. Specific, non-limiting examples of methods that can be used for the detection of a cell surface marker are immunocytochemistry, fluorescence activated cell sorting (FACS), and enzymatic analysis but also RT-PCR and molecular biology methods to detect mRNA of the protein.

As used herein, the term "pluripotent" refers to cells with the ability to give rise to progeny that can undergo differentiation, under appropriate conditions, into cell types that collectively exhibit characteristics associated with cell lineages from the three germ layers (endoderm, mesoderm, and ectoderm). Pluripotent stem cells can contribute to tissues of a prenatal, postnatal or adult organism. A standard art-accepted test, such as the ability to form a teratoma in 8-12-week-old SCID mice, can be used to establish the pluripotency of a cell population. However, identification of various pluripotent stem cell characteristics can also be used to identify pluripotent cells.

In one embodiment of the invention, the pluripotent stem cells are human pluripotent stem cells.

More specifically, human pluripotent stem cells may express at least some, and optionally all, of the markers from the following non-limiting list: SSEA-3, SSEA-4, TRA-I -60, TRA-I -81, TRA-2-49/6E, ALP, Sox 2, E-cadherin, UTF-I, Oct4, Lin28, Rexl, and Nanog.

In one embodiment of the invention, the human pluripotent stem cells are human embryonic stem cells (hESCs) or human induced pluripotent stem cells (hiPSCs).

As used herein, the term "embryonic stem cells" refers to embryonic cells, which are capable of differentiating into cells of all three embryonic germ layers (*i.e*., endoderm, ectoderm and mesoderm), or remaining in an undifferentiated state. For reference only, such cells may comprise cells which are obtained from the embryonic tissue formed after gestation (e.g., blastocyst) before implantation of the embryo (i.e., a pre-implantation blastocyst), extended blastocyst cells (EBCs) which are obtained from a post-implantation/pregastrulation stage blastocyst (see WO2006/040763), embryonic germ (EG) cells which are obtained from the genital tissue of a fetus any time during gestation, preferably before 10 weeks of gestation and other methods with non-fertilized eggs, such as parthenogenesis method.

The embryonic stem cells may be obtained using well-known cell-culture methods. For reference only but not part of the invention, human embryonic stem cells can be isolated from human blastocysts. Human blastocysts are typically obtained from human *in vivo* preimplantation embryos or from *in vitro* fertilized (IVF) embryos. Alternatively, a single cell human embryo can be expanded to the blastocyst stage. For the isolation of human ES cells the zona pellucida is removed from the blastocyst and the inner cell mass (ICM) is isolated by immunosurgery, in which the trophectoderm cells are lysed and removed from the intact ICM by gentle pipetting. The ICM is then plated in a tissue culture flask containing the appropriate medium which enables its outgrowth. Following 9 to 15 days, the ICM derived outgrowth is dissociated into clumps either by a mechanical dissociation or by an enzymatic degradation and the cells are then re-plated on a fresh tissue culture medium. Colonies demonstrating undifferentiated morphology are individually selected by micropipette, mechanically dissociated into clumps, and re-plated. Resulting ES cells are then routinely split every 4-7 days. For further details on methods of preparation human ES cells see for reference only, Thomson et al., [U.S. Pat. No. 5,843,780].

For reference only, commercially available stem cells are also disclosed. Human ES cells can be purchased from the NIH human embryonic stem cells registry (http://escr.nih.gov). Non-limiting examples of commercially available embryonic stem cell lines are BG01, BG02, BG03, BG04, CY12, CY30, CY92, CY10, TE03, TE32, H9, WA09, Roslin Cells (RC6, RC7, RC8, RC9 and RC10) and ESI-017, ESI-035, ESI-049, ESI-051, ESI-053 (Biotime) all disclosed for reference only.

As used herein, the term "induced pluripotent stem cell" refers to a pluripotent stem cell artificially derived from a non-pluripotent cell. A non-pluripotent cell can be a cell of lesser potency to self-renew and differentiate than a pluripotent stem cell. Cells of lesser potency can be, but are not limited to, somatic stem cells, tissue specific progenitor cells, primary or secondary cells. iPSCs have been reproducibly obtained by reprogramming different cell types by forced expression of the OCT4, SOX2, c-MYC and KLF4 transcription factor cocktail or by an alternative combination of factors, substituting KLF4 and c-MYC by or adding NANOG and LIN28, or any methods known from the skilled man to improve reprogramming process (carrying out the use of small molecules such as DNA methyltransferase (DNMT) inhibitors, miRNAs, etc...).

As used herein, the term "reprogramming" refers to the process of changing the fate of a target cell into that of a different cell type, caused by the expression of a small set of factors (or reprogramming factors) in the target cells.

Methods for generating induced pluripotent stem cells based on expression vectors encoding reprogramming factors have been described in the art; see for example WO2007/69666, EP2096169-A1 or WO2010/042490.

Expression vectors for ectopic expression of the reprogramming factors may be, for example, plasmid vector, cosmid vector, bacterial artificial chromosome (BAC) vector, transposon-based vector (such as PiggyBac) or viral vector.

Alternatively, the reprogramming factors, for example, Oct4, Sox2, Klf4 and c-Myc, or corresponding coding DNA or RNA, are introduced into the target cells without integration of exogenous genetic material in the host DNA, i.e. without introduction of the nucleotide sequence in the cell's genome. An expression vector such as a plasmid vector can be delivered into said cells for ectopic expression of the reprogramming factor, in the form of naked DNA. Alternatively, RNAs coding for said reprogramming factors either chemically modified or not, can be introduced into the cells to reprogram them (see for example Warren L, et al, 2010, Cell Stem Cell. Nov 5;7(5):618-30). Other expression vectors have been described for example in WO 2009115295.

In one embodiment, the hiPSCs are derived from cells obtained from a healthy subject. In another embodiment, the hiPSCs are derived from cells obtained from a subject with a liver disease such as an inherited metabolic liver disease and the hepatocyte-like cells display a disease phenotype.

Alternatively, a population of hepatocyte lineage cells of interest may be obtained from the differentiation of multipotent cells, such as mesenchymal stem cells, on a matrix for hepatic differentiation constituted of a laminin of the invention.

As used herein, the term "multipotent" refers to cells capable of differentiating into at least two terminally differentiated cell types.

As used herein, the term "mesenchymal stem cells" generally refers to stromal cells found in a differentiated (specialized) tissue and that are capable of making identical copies of themselves (self-renewal) for the lifetime of the organism and have multipotent differentiation potential (such as differentiation into osteoblasts, adipocytes, chondroblasts). Preferably, human mesenchymal stem cells that can be used in the context of the present invention thus include any suitable human multipotent stem cells (*i.e.,* cells with an ability for self-renewal and for multipotency) derived from any suitable tissue using any appropriate isolation method. For example, human mesenchymal stem cells that can be used in the methods of the present invention include, but are not limited to, adult multilineage inducible (MIAMI) cells (D'Ippolito et al., J. Cell Sci., 2004, 117: 2971-2981), MAPC (also known as MPC) (Reyes al., Blood, 2001, 98: 2615-2625), cord blood derived stem cells (Kögler G et al., J. Exp. Med., 2004, 200(2): 123-135), mesoangioblasts (Sampaolesi M et al., Nature, 2006, 444(7119): 574-579; Dellavalle A et al., Nat. Cell Biol., 2007, 9: 255-267), and amniotic stem cells (De Coppi P et al., Nat Biotechnol 2007, 25:100-106). Furthermore, umbilical cord blood banks (e.g., Etablissement Français du Sang, in France) provide secure and easily available sources of such cells for transplantation.

Alternatively, a population of hepatocyte lineage cells may be obtained from the differentiation of cells isolated from adult human livers (e.g. hepatocyte progenitor cells) on a matrix for hepatic differentiation constituted of a laminin. Still alternatively, a population of hepatocyte lineage cells of interest may be obtained from the conversion of somatic cells such as fibroblasts on a matrix for hepatic differentiation constituted of a laminin.

As used herein, the terms "hepatocyte lineage cells" or "hepatocyte-like cells" (HLCs) refer to cells obtained by differentiating pluripotent cells, endodermal cells or other kind of cells such as multipotent cells in the manner described. The differentiated cells have at least one of a variety of distinguishing phenotypic characteristics of known hepatocyte progenitors, hepatoblasts, foetal, neonates, mature, adult and fully-matured hepatocytes, as provided later in this disclosure. By the use of this term, no particular limitation is implied with respect to cell phenotype, cellular markers, cell function, or proliferative capacity, except where explicitly required. Hepatocyte lineage cells express hepatic markers including but not limited to hepatocyte nuclear factor 4alpha (HNF4α), albumin (ALB), alpha-fetoprotein (AFP), cytochrome P450 and cytokeratin 19 (CK19).

In a particular embodiment, the population of human hepatocyte-like cells is not overexpressing a gene. Preferably, these populations of cells do not overexpress factors involved in hepatic differentiation. Examples of factors involved in hepatic differentiation include without limitation, homeobox transgene HEX, HNF-4, HGF, FGF4, OSM, activin, TGF-β, FOXA2, FGF2, HNF1alpha, HNF1-beta, HNF6, HNF3 beta, SOX17, FOXa3, Foxa1, GATA4, ATF5, PROX1, CEBPalpha or any others genes involved in liver development (i.e. endoderm induction, hepatic specification hepatic maturation, hepatic maintenance, hepatocyte proliferation factors).

The population of human hepatocyte-like cells is not overexpressing the HEX transgene.

As used herein the term "overexpression" refers to the expression of a gene product (RNA or protein) in greater-than-normal amounts. Technics to overexpress a gene or protein are well known in the state of the art and include without limitation, microinjection of mRNA, injection, protein transfection or transfection of an expression vector.

### Methods for determining the expression level of the biomarkers of the invention

Determination of the expression level of markers including hepatic markers (e.g. *HNF4α*, *ALB, AFP, CK19* genes) may be performed by a variety of techniques. Generally, the expression level as determined is a relative expression level. For example, the determination comprises contacting the biological sample with selective reagents such as probes or ligands, and thereby detecting the presence, or measuring the amount, of nucleic acids or polypeptides of interest originally in said biological sample. Contacting may be performed in any suitable device, such as a plate, microtiter dish, test tube, well, glass, column, and so forth. In specific embodiments, the contacting is performed on a substrate coated with the reagent, such as a nucleic acid array or a specific ligand array. The substrate may be a solid or semi-solid substrate such as any suitable support comprising glass, plastic, nylon, paper, metal, polymers and the like. The substrate may be of various forms and sizes, such as a slide, a membrane, a bead, a column, a gel, etc. The contacting may be made under any condition suitable for a detectable complex, such as a nucleic acid hybrid or an antibody-antigen complex, to be formed between the reagent and the nucleic acids or polypeptides of the biological sample.

In a particular embodiment, the expression level of the biomarkers genes may be determined by determining the quantity of mRNA.

Methods for determining the quantity of mRNA are well known in the art. For example, the nucleic acid contained in the cells of interest such as the HLCs of the invention is first extracted according to standard methods, for example using lytic enzymes or chemical solutions or extracted by nucleic-acid-binding resins following the manufacturer's instructions. The extracted mRNA is then detected by hybridization (e.g., Northern blot analysis) and/or amplification (e.g., RT-PCR). Quantitative or semi-quantitative RT-PCR is preferred. Real-time quantitative or semi-quantitative RT-PCR is particularly advantageous.

In another embodiment, the expression level of the biomarkers genes may be determined by determining of the quantity of protein encoded by said genes.

Such methods comprise contacting the biological sample with a binding partner capable of selectively interacting with the protein present in said sample. The binding partner is generally an antibody that may be polyclonal or monoclonal, preferably monoclonal. For example, the level of a biomarker protein such as HNF4α, ALB, AFP and CK19 may be measured by using standard electrophoretic and immunodiagnostic techniques, including immunoassays such as competition, direct reaction, or sandwich type assays. Such assays include, but are not limited to, Western blots; agglutination tests; enzyme-labeled and mediated immunoassays, such as ELISAs; biotin/avidin type assays; radioimmunoassays; Immunoelectrophoresis; immunoprecipitation.

The cell culture conditions may include one or more additional components to provide a supportive environment during directed differentiation into the target differentiated cell type (e.g., hepatocyte lineage cells). The methods for obtaining a population of differentiated target cells additionally include a step of inducing differentiation. Inducing differentiation may be accomplished by changing the composition of growth factors in the culture medium at one or more stages of differentiation as decribed below.

Accordingly, in a second aspect, the invention relates to a method for inducing human hepatic differentiation comprising the steps of:
(i) providing a population of human definitive endoderm (DE) cells, and
(ii) culturing the population on a support coated with a human laminin in a hepatic induction medium to produce a population of human hepatocyte-like cells.

The invention also relates to a method for obtaining human hepatocyte-like cells comprising the steps of:
(i) providing a population of human DE cells, and
(ii) culturing the population on a support coated with a human laminin in a hepatic induction medium to produce a population of human hepatocyte-like cells.

As used herein, the term "definitive endoderm cells" refers to cells expressing characteristic biochemical markers, including but not limited to Sox 17 and FoxA2 and not expressing nanog.

As used herein, the terms "hepatoblast-like cells" (HB) or "hepatic progenitor cells" are interchangeably and refer to cells expressing characteristic biochemical markers, including but not limited to hepatocyte nuclear factor 4alpha (HNF4α), cytokeratin 19 (CK19) and cytochrome P450 3A7 (CYP3A7), and not expressing or substantially not expressing alpha-fetoprotein (AFP) and not expressing albumin (ALB), alpha-1 antitryspin (AAT), cytochrome P450 3A7 (CYP3A7) and uridine diphosphate (UDP)-glucuronosyl transferase 1A1 (UGT1A1).

As used herein, the term "substantially not" refers to a population of hepatocyte-like cells which express a low level of a protein of interest such as AFP. Accordingly, such cells may only express a low quantity or amount of AFP protein undetectable by ELISA, such as several picogramms (pg) which is below the limit of detection (LOD) for AFP by ELISA. However, it should be further noted that a low quantity of AFP mRNA may be detected by RT-PCR (but such quantity is insufficient for detecting *in fine* expression of AFP protein).

As used herein, the expressions "hepatic induction medium" or "culture medium inducing hepatic differentiation" refer to a culture medium that is capable of inducing differentiation of definitive endoderm into hepatocyte-like cells (and thus capable of inducing the expression of hepatic markers such as HNF4α, CK19 and CYP3A7).

As used herein, the term "culture medium" refers to any medium capable of supporting the growth and the differentiation of definitive endoderm cells into hepatic progenitor cells. Preferred media formulations that will support the growth and the differentiation of definitive endoderm cells into hepatic progenitor cells include chemically defined medium (CDM).

As used herein, the term "chemically defined medium" (CDM) refers to a nutritive solution for culturing cells which contains only specified components, preferably components of known chemical structure. A chemically defined medium is a serum-free and feeder-free medium. As used herein, "serum-free" refers to a culture medium containing no added serum. As used herein, "feeder-free" refers to culture medium containing no added feeder cells.

The culture medium used by the invention may be a water-based medium that includes a combination of substances such as salts, nutrients, minerals, vitamins, amino acids, nucleic acids, proteins such as cytokines, growth factors and hormones, all of which are needed for cell survival. For example, a culture medium according to the invention may be a synthetic tissue culture medium such as the RPMI (Roswell Park Memorial Institute medium) or the CMRL-1066 (Connaught Medical Research Laboratory) for human use, supplemented with the needed additives if required as is further described below (Section Examples) such as B27. The B-27 supplement (Invitrogen) contains, amongst other constituents, SOD, catalase and other anti-oxidants (GSH), and unique fatty acids, such as linoleic acid, linolenic acid, lipoic acids.

The step of culturing DE cells with the hepatic induction medium shall be carried out for the necessary time required for the production of hepatocyte-like cells. The duration of this culture step may be determined easily by one of skill in the art. For instance, during the culture the person skilled in the art can monitor the cultured cells for the absence of at least one of expression of markers only expressed by definitive endoderm (DE) cells (e.g. Sox17 and FoxA2) and/or for the expression of markers specifically expressed by hepatocyte-like cells (e.g. HNF4α, CK19, CYP3A7). When expression of one or several markers specific of DE cells cannot be detected and/or expression of one or several markers specific of hepatocyte-like cells is detected, culturing with the hepatic induction medium can be stopped. Monitoring of these markers can be performed using for instance RT-PCR analysis of RNA extracted from cultured cells with specific primers, immunofluorescence analysis with antibodies specific of the markers and FACS or any method to detect the mRNA corresponding to the proteins.

Typically, the step ii) may be carried out for 3 to 10 days, preferably 6 days.

If necessary, the culture medium of the invention can be renewed, partly or totally, at regular intervals. Typically, the culture medium of the invention can be replaced with fresh culture medium of the invention every other day, for 6 days.

The hepatocyte-like cells produced by the above method may be isolated and/or purified using any suitable method, for example FACS.

The term "FGF family growth factor" as used in connection with the method for obtaining human hepatocyte-like cells refers to any naturally occurring substance (e.g. a protein) capable of stimulating cellular growth, proliferation and cellular differentiation by binding to one fibroblast growth factor receptor (FGFR). By binding to one FGFR, the substance increases for example the tyrosine phosphorylation of said receptor.

In one embodiment of the invention, the hepatic induction medium is a chemically defined medium comprising bone morphogenetic protein (BMP) and a fibroblast growth factor (FGF).

In one embodiment of the invention, the hepatic induction medium is a chemically defined medium comprising bone morphogenetic protein 4 (BMP4) and a FGF.

In another embodiment, the hepatic induction medium is a chemically defined medium comprising BMP2 and a FGF.

In another embodiment of the invention, the hepatic induction medium is a chemically defined medium comprising DMSO (dimethylsulfoxide), KOSR (knockout serum replacement), HGF (Hepatocyte Growth Factor), Sodium butyrate. In a particular embodiment, the hepatic induction medium is a chemically defined medium comprising DMSO and KOSR. In another particular embodiment, the hepatic induction medium is a chemically defined medium comprising HGF and sodium butyrate.

Typically, DMSO is added to the culture medium of the invention in a concentration of about 0.5 to about 5%, preferably about 1%. DMSO can be purchased from Sigma. Typically, KOSR is added to the culture medium of the invention in a concentration of about 5% to about 30%, preferably, 20%. KOSR can be purchased from Life Technologies or Thermofisher.

Typically, HGF is added to the culture medium of the invention in a concentration of about 1 ng/mL to about 25 ng/mL, preferably at about 10 ng/mL. HGF can be purchased from R&D Systems. Typically, sodium butyrate is added to the culture medium of the invention in a concentration of about 1 to about 10 mM, preferably at about 2.5 mM. Sodium butyrate can be purchased from Sigma.

In a preferred embodiment of the invention, the hepatic induction medium is a chemically defined medium comprising bone morphogenetic protein 4 (BMP4) and family growth factor 10 (FGF10). The naturally occurring human FGF10 protein has an amino acid sequence as shown in Uniprot Accession number 015520. Typically, FGF10 is added to the culture medium of the invention in a concentration ranging from 1 to 50 ng/ml, preferably at about 10 ng/ml. FGF10 can be purchased from Peprotech or Miltenyi Biotec.

The naturally occurring human BMP4 protein has an amino acid sequence as shown in Uniprot Accession number P12644. Typically, BMP4 is added to the culture medium of the invention in a concentration ranging from 1 to 50 ng/ml, preferably at about 10 ng/ml. BMP4 can be purchased from R&D systems.

The naturally occurring human BMP2 protein has an amino acid sequence as shown in Uniprot Accession number P12643. Typically, BMP2 is added to the culture medium of the invention in a concentration ranging from 1 to 200 ng/ml, preferably at about 10 ng/ml. BMP2 can be purchased from R&D systems or Thermofisher.

In another embodiment of the invention, the hepatic induction medium is a chemically defined medium comprising bone morphogenetic protein 4 (BMP4) and FGF2 (also known as basic fibroblast growth factor).

The naturally occurring human FGF2 protein has an amino acid sequence as shown in Uniprot Accession number P09038. Typically, FGF2 is added to the culture medium of the invention in a concentration ranging from 1 to 50 ng/ml, preferably at about 10 ng/ml. FGF2 can be purchased from Peprotech or Miltenyi Biotec.

In some embodiments of the invention, culturing the population of DE cells may additionally include passaging the population of DE cells prior to or during the process of differentiation. The process of passaging the population of cells may be repeated one or more times, and may include dissociating cells supported by the attachment matrix, diluting the dissociated cells in media. Accordingly the step ii) may further comprise a step of passaging at least one time and/or cell counting.

In some embodiments of the invention, to increase clonogenicity, cells may be treated with a ROCK inhibitor 4 hours prior to dissociation and 24 hours post plating.

As used herein, the term "Rho-associated protein kinase (ROCK) inhibitor" refers to a compound (natural or synthetic) which inhibits ROCK1 and/or ROCK2 activity such as kinase activity.

In a particular embodiment of the invention, the ROCK inhibitor is Y27632 (Watanabe et al. Nature Biotechnology 25, 681 - 686 (2007)).

Typically, the concentration of Y27632 in the culture medium may be from 1 to 100 ng/ml, preferably about 10 ng/ml.

In one embodiment of the invention, said laminin is selected from the group consisting of LN-111, LN-211, LN-332, LN-411, LN-421, LN-511 and LN-521.

The laminin is a human laminin. In one embodiment of the invention, said laminin is a human recombinant laminin.

In one embodiment of the invention, human laminin is coated to the support such as a plate from 0.5 to 50 micrograms per milliliter (µg/mL), preferably from 1 to 10 µg/mL, more preferably at 5 µg/mL.

The support is typically a surface in a culture vessel.

In one embodiment of the invention, the support is selected from the group consisting of a plate, a slide, a flask, and the like. In a preferred embodiment of the invention, the support has at least a portion of a surface coated with a matrix of the invention such as human recombinant LN-111 or human recombinant LN-521.

The invention relates to a method for inducing human hepatic differentiation comprising the steps of culturing a population of human pluripotent or multipotent cells on a support coated with human laminin in an endoderm induction medium to produce a population of human definitive endoderm (DE) cells, and wherein said method does not comprise the overexpression of homeobox transgene HEX in said population of cells.

The invention also relates to a method for inducing human hepatic differentiation comprising the steps of:
(i) culturing a population of human definitive endoderm (DE) cells on a support coated with human laminin in a hepatic induction medium to produce a population of human hepatoblast-like cells, and
(ii) optionally culturing said population of human hepatoblast-like cells on a support coated with human laminin in a hepatic maturation medium to produce a population of hepatocyte-like cells, preferably human fetal hepatocyte-like cells,
and wherein said method does not comprise the overexpression of homeobox transgene *HEX* in said population of cells.

As used herein, the expressions "endoderm induction medium" or "culture medium inducing endoderm differentiation" refer to a culture medium that is capable of inducing differentiation of pluripotent stem cells into definitive endoderm cells (and thus capable of inducing the expression of endoderm markers such as Sox 17 and FoxA2).

In a preferred embodiment of the invention, the endoderm induction medium is a chemically defined medium comprising at least Activin A and optionally WNT3A.

In another embodiment of the invention, the endoderm induction medium is a chemically defined medium further comprising CHIR99021.

Activin A is well known in the art and is a dimeric polypeptide which exerts a range of cellular effects via stimulation of the Activin/Nodal pathway (Vallier et al., Cell Science 118:4495-4509 (2005)). The naturally occurring human activin A protein has an amino acid sequence as shown in GeneBank Accession number NP_002183. Activin is readily available from commercial sources (e.g. Stemgent Inc. MA USA or Miltenyi Biotec).

Typically, the concentration of Activin A in the culture medium may be from about 10 to about 1000 ng/ml, preferably about 100 ng/ml.

The naturally occurring human WNT3A protein has an amino acid sequence as shown in Uniprot Accession number P56704. Typically, the concentration of WNT3A in the culture medium may contain 10 to 100 ng/ml WNT3A, preferably about 50 ng/ml. WNT3A can be purchased from Miltenyi Biotec.

CHIR99021 is an inhibitor of GSK3 that activates the WNT/β-Catenin pathway. CHIR99021 can be purchased from Tocris Bioscience, Stemgent.

Typically, the concentration of CHIR99021 in the culture medium is from about 1µM to about 10 µM, preferably about 3µM.

The step of culturing pluripotent stem cells with the endoderm induction medium shall be carried out for the necessary time required for the production of definitive endoderm (DE). The duration of this culture step may be determined easily by one of skill in the art. For instance, during the culture the person skilled in the art can monitor the cultured cells for the expression of markers specifically expressed by definitive endoderm (DE) (e.g. Sox 17 and FoxA2). When expression of one or several markers specific of DE cells is detected, culturing with the hepatic induction medium can be stopped. Monitoring of these markers can be performed using for instance RT-PCR analysis of RNA extracted from cultured cells with specific primers, immunofluorescence analysis with antibodies specific of the markers, ELISA and FACS or any method to detect the RNA/protein/activity corresponding to the specific marker.

Typically, the step ii) may be carried out for 1 to 10 days, preferably 5 days.

If necessary, the culture medium of the invention can be renewed, partly or totally, at regular intervals. Typically, the culture medium of the invention can be replaced with fresh culture medium of the invention every other day, for 5 days.

The hepatocyte-like cells produced by the above method may be isolated and/or purified using any suitable method, for example FACS.

In one embodiment of the invention, said laminin is selected from the group consisting of LN-111, LN-211, LN-332, LN-411, LN-421, LN-511 and LN-521.

The laminin is a human laminin. In one embodiment of the invention, said laminin is a human recombinant laminin.

In one embodiment of the invention, human laminin is coated to the support such as a plate from 0.5 to 50 micrograms per milliliter (µg/mL), preferably from 1 to 10 µg/mL, more preferably at 5 µg/mL.

The support is typically a surface in a culture vessel.

In one embodiment of the invention, the support is selected from the group consisting of a plate, a slide, a flask, and the like. In a preferred embodiment of the invention, the support has at least a portion of a surface coated with a matrix of the invention such as human recombinant LN-111 and/or human recombinant LN-521.

The invention further relates to a method for inducing human hepatic differentiation comprising the steps of:
(i) culturing a population of human pluripotent or multipotent cells on a support coated with human laminin in an endoderm induction medium to produce a population of human definitive endoderm (DE) cells,
(ii) culturing said population of human DE cells on a support coated with human laminin in a hepatic induction medium to produce a population of human hepatoblast-like cells, and
(iii) optionally culturing said population of human hepatoblast-like cells on a support coated with human laminin in a hepatic maturation medium to produce a population of hepatocyte-like cells, preferably human fetal hepatocyte-like cells
and wherein said method does not comprise the overexpression of homeobox transgene *HEX* in said population of cells.

As used herein, the terms "fetal hepatocyte-like cells" or "differentiated hepatoblasts" are used herein interchangeably and refer to cells expressing characteristic biochemical markers, including but not limited to HNF4α, CYP3A7, AFP and CK19, and may expressed some mature hepatic proteins, including but not limited to ALB, AAT, UGT1A1, and cytochrome P450 3A4 (CYP3A4).

As used herein, the expressions "hepatic maturation medium" or "culture medium stimulating hepatic maturation" refer to a culture medium that is capable of inducing maturation of hepatoblast-like cells to fetal hepatocyte-like cells.

In a preferred embodiment of the invention, the hepatic induction medium is a chemically defined medium comprising HGF and OSM.

As used herein, the term "hepatic growth factor" (HGF) refers to a growth factor which regulates cell growth, cell motility, and morphogenesis by activating a tyrosine kinase signaling cascade after binding to the proto-oncogenic c-MET receptor. The naturally occurring human HGF protein has an amino acid sequence as shown in Uniprot Accession number P14210.

Typically, HGF is added to the hepatic maturation medium at a concentration ranging from 1 to 100 ng/ml, preferably from 5 to 50 ng/ml, and even more preferably about 20 ng/ml. HGF can be purchased from Peprotech.

As used herein, the term "oncostatin M" (OSM) refers to a cytokine which inhibits the proliferation of a number of tumor cell lines. The naturally occurring human OSM protein has an amino acid sequence as shown in Uniprot Accession number P13725.

Typically, Oncostatin M is added to the hepatic maturation medium at a concentration ranging from 1 to 100 ng/ml, preferably from 5 to 50 ng/ml, and even more preferably about 20 ng/ml. OSM can be purchased from Miltenyi Biotec.

The step of culturing hepatocyte-like cells with the hepatic maturation medium shall be carried out for the necessary time required for the production of fetal hepatocyte-like cells. The duration of this culture step may be determined easily by one of skill in the art. For instance, during the culture the person skilled in the art can monitor the cultured cells for the absence of expression of specific markers of pluripotent stem cells (e.g. Sox 2 and Nanog), absence of expression of specific markers of definitive endoderm (DE) (e.g. Sox 17 and FoxA2), expression of specific markers of hepatoblasts and/or for the expression of markers specifically expressed by fetal hepatocyte-like cells (e.g. AFP and ALB). When expression of one or several markers specific of fetal hepatocyte-like cells is detected, culturing with the hepatic maturation medium can be stopped. Monitoring of these markers can be performed using for instance RT-PCR analysis of RNA extracted from cultured cells with specific primers, immunofluorescence analysis with antibodies specific of the markers, ELISA and FACS or any method to detect the RNA/protein/activity corresponding to the specific marker.

Typically, the step ii) may be carried out for 3 to 60 days, preferably 30 days.

If necessary, the culture medium of the invention can be renewed, partly or totally, at regular intervals. Typically, the culture medium of the invention can be replaced with fresh culture medium of the invention every other day, for 30 days.

The fetal hepatocyte-like cells produced by the above method may be isolated and/or purified using any suitable method, for example FACS.

In one embodiment of the invention, said laminin is selected from the group consisting of LN-111, LN-211, LN-332, LN-411, LN-421, LN-511 and LN-521.

The laminin is a human laminin. In one embodiment of the invention, said laminin is a human recombinant laminin.

In one embodiment of the invention, human laminin is coated to the support such as a plate from 0.5 to 50 micrograms per milliliter (µg/mL), preferably from 1 to 10 µg/mL, more preferably at 5 µg/mL.

The support is typically a surface in a culture vessel.

In one embodiment of the invention, the support is selected from the group consisting of a plate, a slide, a flask, and the like. In a preferred embodiment of the invention, the support has at least a portion of a surface coated with a matrix of the invention such as human recombinant LN-111 and/or human recombinant LN-521.

### Populations of human hepatocyte-like cells which are not part of the invention and pharmaceutical compositions comprising them

In another aspect, the disclosure relates to a population of human hepatocyte-like cells obtained by a method as defined above.

The disclosure also relates to a population of human hepatoblast-like cells obtained by a method as defined above, wherein said cells express hepatocyte nuclear factor 4alpha (HNF4α) and do not express or substantially not express alpha-fetoprotein (AFP).

The disclosure relates to a population of human hepatoblast-like cells obtained by a method as defined above, wherein said cells express HNF4α, cytokeratin 19 (CK19) and cytochrome P450 3A7 (CYP3A7) and do not express or substantially not express AFP, albumin (ALB), alpha-1 antitryspin (AAT), cytochrome P450 3A4 (CYP3A4), uridine diphosphate (UDP)-glucuronosyl transferase 1A1 (UGT1A1).

In still another aspect, the disclosure relates to a population of human fetal hepatocytes cells obtained by a method as defined above.

The disclosure also relates to a population of human fetal hepatocyte-like cells obtained by a method as defined above, wherein said cells express HNF4α, CK19, CYP3A7, AFP, ALB, AAT and UGT1A1, and cytochrome P450 3A4 (CYP3A4).

In one embodiment, the human hepatocyte-like cells display a normal phenotype.

In another embodiment, the human hepatocyte-like cells display a genetic mutation, in particular genetic mutation that affects key protein within human hepatocyte-like cells and which is associated with a liver disease. It should be noted that, the genetic mutation or defect which is responsible for the disease phenotype may be corrected *in vitro or ex vivo.* Various techniques are available to correct genetic mutations or defects in isolated mammalian cells.

### Genetic modification of human hepatocyte-like cells which are not part of the invention

The term "genetically modified" indicates that the human hepatocyte-like cells comprise a nucleic acid molecule not naturally present in non-modified human hepatic progenitor cells, or a nucleic acid molecule present in a non-natural state in said human hepatic progenitor cells (e.g., amplified). The nucleic acid molecule may have been introduced into said cells or into an ancestor thereof (such as iPS which contains a liver disease associated genetic mutation).

A number of approaches can be used to genetically modify human hepatocyte-like cells, such as virus-mediated gene delivery, non-virus-mediated gene delivery, naked DNA, physical treatments, etc. To this end, the nucleic acid is usually incorporated into a vector, such as a recombinant virus, a plasmid, phage, episome, artificial chromosome, etc.

In a particular embodiment of the disclosure the hepatocyte-like cells (HLCs), or the pluripotent stem cells, are genetically modified using a viral vector (or a recombinant virus) or a non viral method. In this embodiment, the heterologous nucleic acid is, for example, introduced into a recombinant virus which is then used to infect human hepatocyte-like cells (HLCs). Different types of recombinant viruses can be used, in particular lentivirus.

Also disclosed, said lentivirus encodes an immortalizing protein, such as SV40T, hTERT, CDK4, etc.

Said lentivirus may encode a wild-type protein (such a protein usually displaying a disease associated genetic mutation in patients suffering from a liver disease) such as wild-type α1-antitrypsin (AAT) protein or UDP glucuronosyltransferase 1 family, polypeptide A1 (UGT1A1). Other genetic mutations involved in liver diseases have been previously described.

Also disclosed, the nucleic acid sequence encoding the protein of interest (for instance an immortalizing protein or a wild-type protein used for correcting a disease phenotype) is operatively linked to a promoter.

Alternatively, said lentivirus encodes a reporter protein or marker protein. Said marker protein, which may be a fluorescent protein or a cell surface expressed protein, permits the rapid identification and isolation of human hepatocyte-like cells (HLCs) of interest.

The marker protein may, for instance, be selected from the group consisting of:
- a fluorescent protein, in particular the green fluorescent protein (GFP) or its derivates, for instance the enhanced green fluorescent protein, the blue fluorescent proteins (EBFP, EBFP2, Azurite, mKalamal ), the cyan fluorescent proteins (ECFP, Cerulean, CyPet), and the yellow fluorescent proteins (YFP, EYFP, Citrine, Venus, YPet); and
- any cell surface expressed protein which is not naturally expressed by HLCs.

As used herein, the term "operably linked" means that the components described are in a relationship permitting them to function in their intended manner. Thus, a nucleic acid sequence is "operably linked" when placed into a functional relationship with another sequence nucleic sequence. For instance, a promoter is "operably linked" to a coding sequence if the promoter causes the transcription of the coding sequence. Generally, operably linked means the linked nucleic acid sequences are contiguous.

As used herein, the term "promoter" refers to a DNA sequence that determines the site of transcription initiation for an RNA polymerase. A promoter may comprise a RNA polymerase III promoter that can provide high levels of constitutive expression across a variety of cell types and will be sufficient to direct the transcription of a distally located sequence, which is a sequence linked to the 3' end of the promoter sequence in a cell. Suitable promoters include, for example, constitutive, regulated, tissue-specific or ubiquitous promoters, which may be of cellular, viral or synthetic origin.

The promoter may be a constitutive promoter such as human elongation factor-1 alpha (EF-1 alpha), etc.

The promoter may be a liver-specific promoter such as, human 1-antitrypsin, albumin, etc.

The promoter may be an endogenous sequence (constitutive or specific). In that case, the protein of interest is linked to the endogenous promoter by a genome editing technology based on the use of an artificial nuclease (ex: CRISPR/cas, ZFNs, TALENs).

Also disclosed gene modification of human hepatocyte-like cells is performed by genome editing technology to modify the endogenous gene sequence.

Importantly, hepatocyte-lineage cells (HLCs) are the target of disease in a variety of conditions often known as "liver diseases" (also referred as "pathology associated with hepatic damage "). These terms refer to any disease or clinical condition characterized by hepatic damage, injury, dysfunction, defect, or abnormality. Thus, the term encompasses, for example, injuries, degenerative diseases and genetic diseases.

Liver diseases are becoming one of the most common causes of mortality in developing countries. This group of diseases which targets hepatocyte-lineage cells such as hepatocytes which represent the dominant liver cells encompasses inherited metabolic disorders (such as Crigler-Najjar Syndrome type I, Glycogen storage disease, Urea cycle defects, familial hypercholesterolemia, tyrosinemia and Wilson's Disease), chronic liver failure as well as acute liver failure which may be caused by viral infection (in particular infection with HBV or HCV), toxic (alcohol) and drugs, or autoimmune disorder (Autoimmune Chronic Hepatitis, Primary Biliary Cirrhosis, Primary Sclerosing Cholangitis).

Not part of the invention is a pharmaceutical composition comprising the population of human hepatocyte-like cells

The pharmaceutical composition may generally include one or more pharmaceutically acceptable and/or approved carriers, additives, antibiotics, preservatives, adjuvants, diluents, solvant and/or stabilizers. Such auxiliary substances can be water, saline, glycerol, ethanol, wetting or emulsifying agents, pH buffering substances, or the like. Suitable carriers are typically large, slowly metabolized molecules such as proteins, polysaccharides, polylactic acids, polyglycollic acids, polymeric amino acids, amino acid copolymers, lipid aggregates, or the like. This pharmaceutical composition can contain additional additives such as mannitol, dextran, sugar, glycine, lactose or polyvinylpyrrolidone or other additives such as antioxidants or inert gas, stabilizers or recombinant proteins (e. g. human serum albumin) or vitamins suitable for *in vivo* administration.

As used herein, the term "pharmaceutically acceptable" refers to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

Screening methods which are not part of the invention

In another aspect, the human hepatocyte-like cells obtained from healthy or diseased patients may also be used advantageously for screening applications in the pharmaceutical industry.

Such screening tests can be used to search for new drugs with clinical applications or for toxicology tests evaluation of hepatotoxicity of compounds such as pharmaceutical candidate compounds. The human hepatocyte-like cells may for instance be useful for generating cellular models of liver diseases as described above.

Accordingly, the disclosure provides a method of screening for a compound useful in the treatment of a liver disease comprising the steps of:
(a) contacting a population of human hepatoblast-like cells or fetal hepatocyte-like cells produced by a method of the invention with a test compound, and
(b) determining the effect of the test compound on said human hepatoblast-like cells or fetal hepatocyte-like cells.

A further aspect of the disclosure relates to a method for screening compounds having a hepatoprotective or hepatotoxic or hepatoproliferative effect wherein said method comprises the steps of:
(a) contacting a population of human hepatoblast-like cells or fetal hepatocyte-like cells produced by a method of the invention with a test compound, and
(b) comparing the survival of the cells of step a) to that of a population of said cells as defined above cultured in the absence of said test compound.

The term "hepatotoxic" refers to a compound which provokes a decrease in the survival of hepatic progenitor cells. A compound is deemed to have a hepatotoxic effect if the number of viable cells cultured in the presence of said compound is lower than the number of viable cells cultured in the absence of said compound.

The term "hepatoprotective" refers to a compound which results in an increase survival of the hepatic progenitor cells. A compound is deemed to have a hepatoprotective effect if the number of viable cells cultured in the presence of said compound is higher than the number of viable cells cultured in the absence of said compound.

Typically, the hepatoprotective effect can be assayed in the absence of hepatotrophic factors. Alternatively, the hepatoprotective effect can be assayed in the presence of a known hepatotoxic drug. Known hepatotoxic drugs include, but are not limited to amiodarone, methotrexate, nitrofurantoin.

The term "hepatoproliferative" refers to a compound which results in an increase proliferation of the hepatic progenitor cells. A compound is deemed to have a hepatoproliferative effect if the number of proliferative cells cultured in the presence of said compound is higher than the number of viable cells cultured in the absence of said compound. Typically, the hepatoproliferative effect can be assayed in the absence of growth factors.

Test compounds which are not part of the invention

In one embodiment, the test compound may be selected from the group consisting of peptides, proteins, peptidomimetics, small organic molecules, aptamers or nucleic acids. For example, the test compound according to the invention may be selected from a library of compounds previously synthesised, or a library of compounds for which the structure is determined in a database, or from a library of compounds that have been synthesised *de novo.*

In a particular embodiment, the test compound may be selected from small organic molecules. As used herein, the term "small organic molecule" refers to a molecule of size comparable to those organic molecules generally used in pharmaceuticals. The term excludes biological macromolecules (e.g.; proteins, nucleic acids, etc.); preferred small organic molecules range in size up to 2000 Da, and most preferably up to about 1000 Da. In another embodiment, the test compound may be selected from the group consisting of a nucleic acids library, including but not limited to shRNA, miRNA, mRNA.

### Therapeutic methods and uses which are not part of the invention

One major field of application is cell therapy or regenerative medicine. Regenerative medicine can be used to potentially cure any disease that results from malfunctioning, damaged or failing tissue by either regenerating the damaged tissues *in vivo* by direct *in vivo* implanting of a pharmaceutical composition comprising hepatocyte-like cells of the invention.

The disclosure also relates to a pharmaceutical composition for treating or for use in treating a liver disease comprising or consisting of or consisting essentially of a population of hepatocyte-like cells as described above and at least one pharmaceutically acceptable excipient.

Another object of the disclosure is a medicament for treating or for use in treating a liver disease comprising or consisting of or consisting essentially of a population of hepatocyte-like cells.

As used herein, the term "consisting essentially of', with reference to a pharmaceutical composition or medicament, means that the at least one compound of the disclosure is the only one therapeutic agent or agent with a biologic activity within said pharmaceutical composition or medicament.

Pharmaceutically acceptable excipients refer to any and all solvents, dispersion media, coatings, antibacterial and antifungal agents, isotonic and absorption delaying agents and the like. In one embodiment, the excipient comprises additives proteins, peptides, amino acids, lipids, and carbohydrates (e.g., sugars, including monosaccharides, di-, tri-, tetra-, and oligosaccharides; derivatized sugars such as alditols, aldonic acids, esterified sugars and the like; and polysaccharides or sugar polymers), which can be present singly or in combination, comprising alone or in combination 1-99.99% by weight or volume.

Exemplary protein excipients include serum albumin such as human serum albumin (HSA), recombinant human albumin (rHA), gelatin, casein, and the like.

Representative amino acid/antibody components, which can also function in a buffering capacity, include alanine, glycine, arginine, betaine, histidine, glutamic acid, aspartic acid, cysteine, lysine, leucine, isoleucine, valine, methionine, phenylalanine, aspartame, and the like. Carbohydrate excipients are also intended within the scope of this invention, examples of which include but are not limited to monosaccharides such as fructose, maltose, galactose, glucose, D-mannose, sorbose, and the like; disaccharides, such as lactose, sucrose, trehalose, cellobiose, and the like; polysaccharides, such as raffinose, melezitose, maltodextrins, dextrans, starches, and the like; and alditols, such as mannitol, xylitol, maltitol, lactitol, xylitol sorbitol (glucitol) and myoinositol. For human administration, preparations should meet sterility, pyrogenicity, general safety and purity standards as required by regulatory offices, such as, for example, FDA Office or EMA.

The disclosure also relates to a population of human cells committed in the hepatocyte lineage but not fully differentiated for use in a method of treatment of the human body. The disclosure also relates to a population of human cells committed in the hepatocyte lineage but not fully differentiated for use in the treatment of a liver disease.

Examples of liver diseases include without limitation, hepatic cirrhosis, hepatic steatosis, non-alcoholic steatohepatitis (NASH), alcoholic hepatitis, fatty liver disease, fatty liver of pregnancy, viral induced-hepatitis A, B, C, D and E, iron overload disorders, hepatic fibrosis, congenital liver disease ( such as type 1 Crigler-Najjar (CN1), Wilson's disease, urea cycle disorders, tyrosinemia, familial hypercholesterolemia, hemophilias, citrullinemia, progressive familial intrahepatic cholestasis, glycogen storage disease), acute liver failure fulminant hepatitis, sub-fulminant hepatitis, liver cancers, hypercholesterolemia.

Such diseases can be induced by environmental factors including without limitation, drugs, toxic mushroom, post-surgical infections, virus, bacteria, and alcohol.

In one embodiment, said population of human hepatoblast-like cells is a population of said cells expressing hepatocyte nuclear factor 4alpha (HNF4α) and do not expressing or substantially not expressing alpha-fetoprotein (AFP) as above-described.

More particularly, an aspect of the disclosure relates to a population of hepatocyte-like cells of the invention for use in the treatment of a liver disease. In one embodiment, said population of human hepatoblast-like cells is a population of said cells expressing hepatocyte nuclear factor 4alpha (HNF4α) and do not expressing or substantially not expressing alpha-fetoprotein (AFP) as above-described.

The disclosure also relates to a method for treating a liver disease comprising the step of administering a pharmaceutically effective amount of a population of hepatocyte-like cells to a subject or a patient in need thereof

As used herein, the term "pharmaceutically effective amount" refers to any amount of a population of human hepatocyte-like cells according to the disclosure (or a pharmaceutical composition thereof) that is sufficient to achieve the intended purpose.

Effective dosages and administration regimens can be readily determined by good medical practice based on the nature of the pathology of the subject, and will depend on a number of factors including, but not limited to, route of administration, the extent of the symptoms of the pathology, the specific pathology and extent of damage or degeneration of the tissue or organ of interest, and characteristics of the subject (e.g., age, body weight, gender, general health, and the like).

For therapy, populations of human hepatocyte-like cells and pharmaceutical compositions may be administered through different routes. The dose and the number of administrations can be optimized by those skilled in the art in a known manner.

In one embodiment, the population of human hepatocyte-like cells, the pharmaceutical composition, the medicament is to be administered locally of systemically.

In one embodiment, the population of human hepatocyte-like cells, the pharmaceutical composition, the medicament is to be administered locally and include without limitation, an injection or an infusion or an implantation of the population of human hepatocyte-like cells, the pharmaceutical composition, the medicament in, around or near the liver, in the liver parenchyma, under the liver Glisson's capsule, under kidney capsule, in the spleen, in the pancreas, in the peritoneum and omental pouch. Preferably, the local administration is an injection or an infusion or an implantation via blood vessels irrigating the liver (portal vein, artery, vein, mesenteric veins).

In another embodiment, the population of human hepatocyte-like cells, the pharmaceutical composition, or the medicament are to be administered in a differentiating environment for the population of human hepatocyte-like cell

Such route of administration can be achieved by surgery procedure, laparoscopic surgery, via a catheter system or an implantation in the peritoneal cavity.

In one embodiment, the population of human hepatocyte-like cells, the pharmaceutical composition, the medicament is to be administered systemically and include without limitation, enteral or parenteral administration.

Examples of formulations adapted to injections or infusion or implantation include, but are not limited to, liquid solutions or suspensions, solid forms suitable for solution in, or suspension in, liquid prior to injection. Examples of injections include, but are not limited to, intravenous, intra-aortic, intraperitoneal, subcutaneous, intramuscular, intradermal, and intraperitoneal injection, or perfusion. In another embodiment, when injected, the population of human hepatocyte-like cells, the pharmaceutical composition or the medicament of the invention is sterile. Methods for obtaining a sterile pharmaceutical composition include, but are not limited to, GMP synthesis (GMP stands for "Good manufacturing practice").

In one embodiment, the population of human hepatocyte-like cells, the pharmaceutical composition or the medicament is encapsulated. Examples of capsules include without limitation, matrigel, hydrogel.

In one embodiment, the population of human hepatocyte-like cells, the pharmaceutical composition or the medicament is to be administered in a sustained-release form. In another embodiment, the population of human hepatocyte-like cells, the pharmaceutical composition or the medicament comprises a delivery system that controls the release of the agent.

In one embodiment, a therapeutically effective amount of the population of human hepatocyte-like cells, the pharmaceutical composition or the medicament is to be administered at least once in the subject's life or several times to obtain and/or to maintain therapeutic benefit in the subject.

In another embodiment, a therapeutically effective amount of the population of human hepatocyte-like cells or the pharmaceutical composition or the medicament ranges from about 1 to about 200 millions cells per kg of body, preferably about 25 millions cells per kg of body.

The term "about" as used herein preceding a figure means plus or less 10% of the value of said figure.

In one embodiment, the subject is affected, preferably is diagnosed with a liver disease.

In one embodiment, the human hepatocyte-like cells may be useful for autologous regenerative therapy of a patient suffering from a liver disease in need of regenerative therapy due to specific disorders or treatments associated to such disorders, including without limitation, type 1 Crigler-Najjar (CN1) due to the deficit in one identified gene that can be replaced *in vitro.*

In one embodiment, the disclosure
relates to the human hepatocyte-like cells of the invention for use as a cell therapy product for implanting into a human patient, as an allogenic graft or, after genetic correction, as an autologous graft (i.e the cells have the same genotype as the subject/patient's cells).

In one embodiment, the disclosure relates to the human hepatocyte-like cells of the invention for use as an auxiliary liver. In one embodiment, the hepatocyte-like cells when administered, localized in the spleen. In one embodiment, the spleen containing the hepatocyte-like cells of the invention is acting as a secondary liver.

In one embodiment, the human hepatocyte-like cells may be useful for bioengineered livers: either by infusing them together with other hepatic-lineage cells into a decellularized liver, by generation of vascularized and functional human liver from human iPSCs by transplantation of liver buds created *in vitro* (Takebe et al Nature 2013 499), by 3D printing or every other method to generate liver tissue.In another embodiment, the human hepatocyte-like cells may be useful for bioartificial livers either by infusing them into extracorporeal devices or embedded them in a biomatrice or hygrogel (such as alginate, silanized hydroxypropyl methylcellulose) before infusion in the body.

The invention will be further illustrated by the following figures and examples. However, these examples and figures should not be interpreted in any way as limiting the scope of the present invention.

### FIGURES

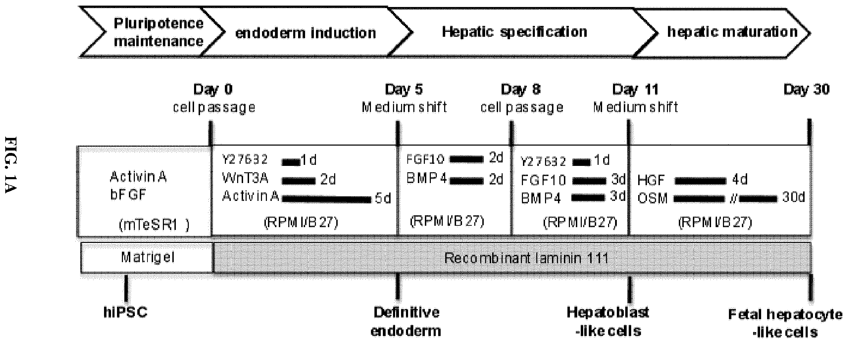

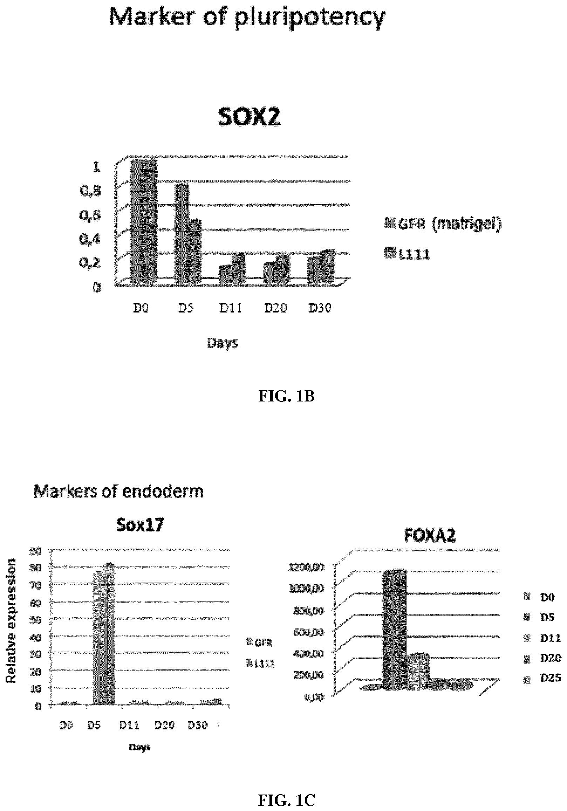

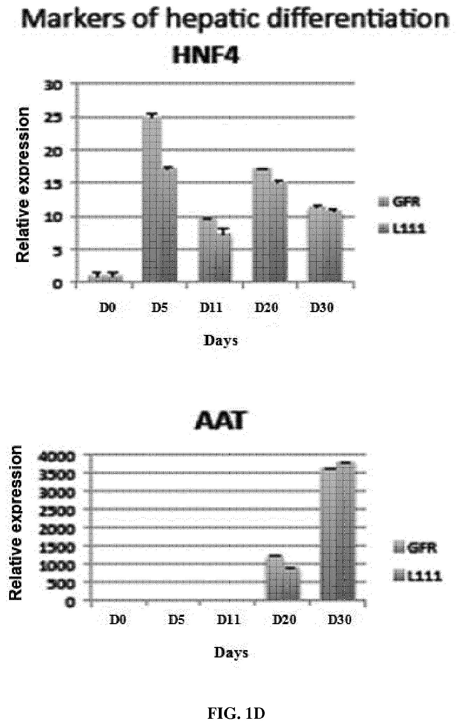

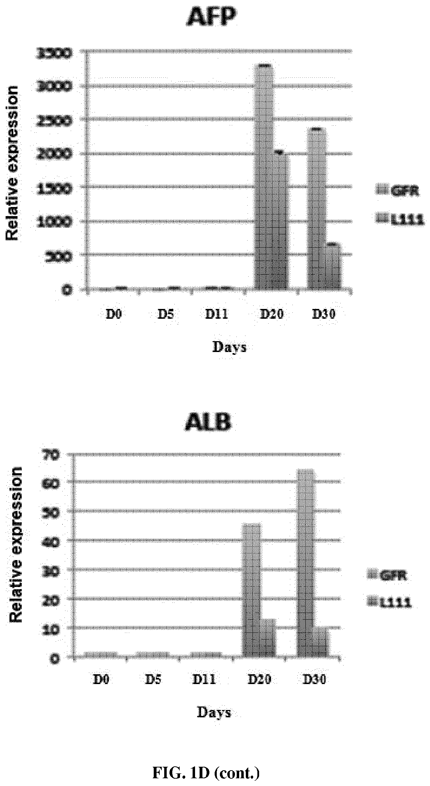

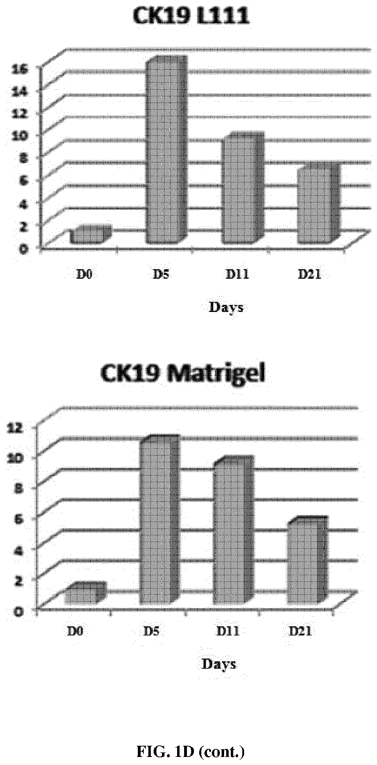

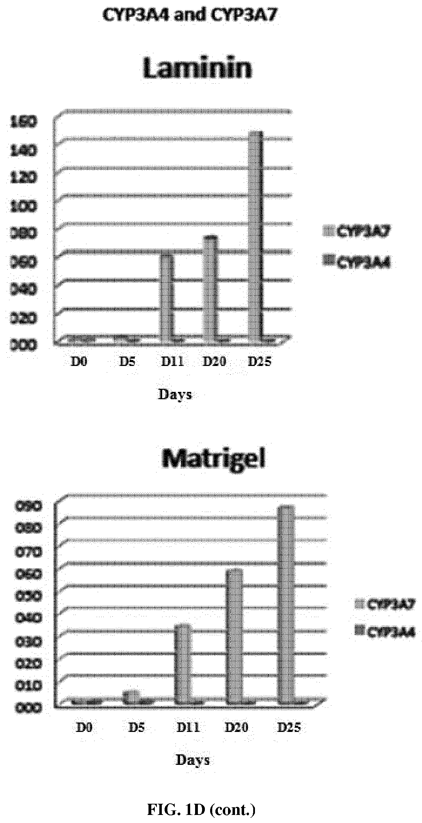

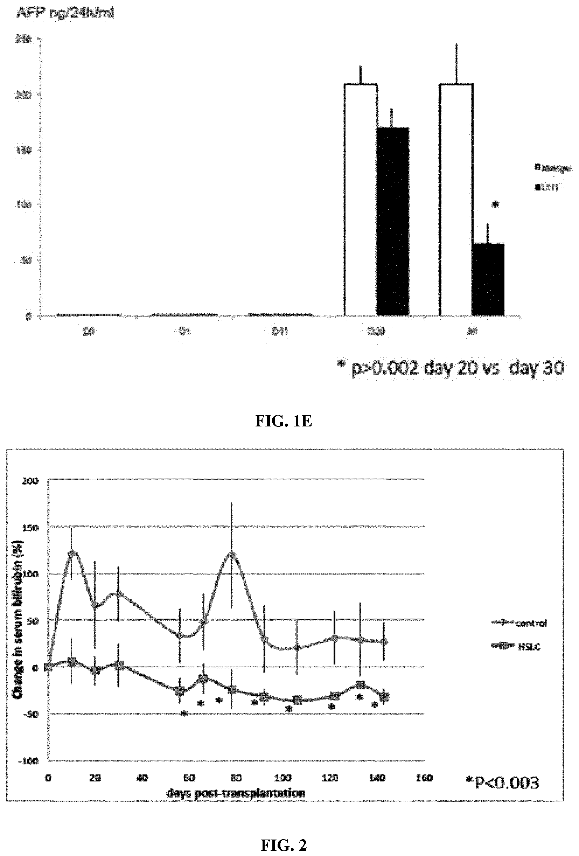

**Figure 1****: Hepatic differentiation of human pluripotent stem cells into LN111-coated dishes.**
   (**A**) Protocol to differentiate pluripotent human cells into hepatocyte-like cells. **(B-D)** Hepatic differentiation was monitored over time by RT-qPCR analyses for mRNA expression of pluripotency marker (panel B, SOX2), of endoderm markers (panel C, SOX17, FOXA2) and hepatic markers (panel D, HNF4a, AAT, AFP, ALB, CK19, CYP3A4, CYP3A7, UGT1A1). **(E)** Secretion of hepatic proteins AFP in cell supernatants by ELISA. Data represent mean ± SEM. LN111: results with hiPSC differentiated onto LN111-coated dishes; Matrigel: results with hiPSC differentiated onto Matrigel^{™}-coated dishes.
**Figure 2****: Transplantation of HLCS into the livers of Gunn rats. Serum bilirubin levels in Gunn rats.**
   Bilirubinemia was monitored over time in tacrolimus-immunosuppressed Gunn rats receiving IDHBs 24 hours after a two-third partial hepatectomy. Control rats received the same surgical procedures but were not transplanted.
**Figure 3****: Hepatic differentiation of human pluripotent stem cells into LN521-coated dishes.**
   Hepatic differentiation was monitored over time by RT-qPCR analyses for mRNA expression of endoderm markers (SOX17) and hepatic markers (HNF4a, AAT, AFP, ALB and CK19).
**Figure 4****: Hepatic differentiation of iPSC cells into LN111-coated dishes.**
   Hepatic differentiation was monitored over time by RT-qPCR analyses for mRNA expression of endoderm markers (Nanog, OCT4, FOXA2, SOX17).
**Figure 5****: Hepatic differentiation of iPSC cells into LN111-coated dishes.**
   Hepatic differentiation was monitored over time by RT-qPCR analyses for mRNA expression of hepatic markers (HNF4a, CYP7A1, AFP and CK19).
**Figure 6****: Hepatic differentiation of iPSC cells into LN111-coated dishes.**
   Hepatic differentiation was monitored over time by RT-qPCR analyses for mRNA expression of hepatic markers (ALB, AAT).
**Figure 7****: Hepatic differentiation of iPSC cells into LN111 or LN521-coated dishes.**
   Hepatic differentiation was monitored over time by RT-qPCR analyses for mRNA expression of hepatic markers (HNF4a, AAT, AFP and ALB).
**Figure 8****: Hepatic differentiation of hESCs into LN111 or LN521-coated dishes with or without CHIR99021.**
   Hepatic differentiation was monitored over time by RT-qPCR analyses for mRNA expression of hepatic markers (HNF4a, CK19, AFP and CYP3A7).
**Figure 9****: Influence of cell seeding on hepatic differentiation of hESCs into LN111 or LN521-coated dishes with or without CHIR99021.**
   Hepatic differentiation was monitored over time by RT-qPCR analyses for mRNA expression of hepatic markers (HNF4a, CK19, AFP and ALB).
**Figure 10****: Transplantation of fresh or frozen iPS hepatic cells into the livers of Gunn rats.**
   Bilirubinemia was monitored over time in tacrolimus-immunosuppressed Gunn rats receiving IDHBs 24 hours after a two-third partial hepatectomy. Control rats received the same surgical procedures but were not transplanted.
**Figure 11****: Detection of hepatic cells in the spleen or the liver of Gunn rats.**
   Immunohistochemical analysis of UGT1A1 in the liver and spleen of tacrolimus-immunosuppressed Gunn rats receiving IDHBs 24 hours after a two-third partial hepatectomy.

### REFERENCE EXAMPLE

### 1: USE OF LN-111 AS A MATRIX FOR DIFFERENTIATING PLURIPOTENT CELLS INTO HEPATOCYTE LINEAGE CELLS AND USE OF HEPATOBLAST-LIKE CELLS-LIKE CELLS THUS OBTAINED IN THERAPY.

### Material & Methods

This study was performed in agreement with the French and European regulations.

**Maintenance of hIPSC:** The BBHX8 hiPSC [46] lines was maintained on Matrigel (BD Biosciences, San Jose, CA, USA)-coated culture wells in mTeSR1 (Stemcell Technologies, Vancouver, BC, Canada) at 37 °C in a 5% CO2 incubator with daily medium changes. Cells were passaged every 5-7 day with Gentle Cell Dissociation Reagent (Stemcell Technologies, Vancouver, BC, Canada).

**Hepatic Differentiation in vitro:** Hepatic differentiation of human pluripotent stem cells was performed following a three-step protocol based on previous studies [16, 20, 40] with some modifications. First, for definitive endoderm differentiation, cells were harvested using TrypLE (Life Technologies, Carlsbad, CA, USA), counted using ADAM automatic cell counter (Labtech, Palaiseau, France) and then plated into plates (Costar; Corning Life Sciences, Acton, MA) precoated either with 5 µg/ml laminin 111 (Biolamina, Sundbyberg, Sweden) or 2 mg/ml Matrigel (BD Biosciences, San Jose, CA, USA) at a cell density of 75×10³ cells/cm². They were culture in RPMI 1640 supplemented with supplemented with B27 serum-free supplement (Life technologies, Carlsbad, CA, USA) (RPMI/B27), 100 ng/ml Activin A (Miltenyi Biotec, Paris, France), 50 ng/ml WnT3A (R&D systems, Minneapolis, MN, USA), 10µM rock inhibitor (Stemcell Technologies, Vancouver, BC, Canada), and optionally CHIR 99021 for 1 day. Rock inhibitor and Wnt3A were omitted from the medium on the following 2 days and 3 days, respectively. The following 4 days, cells were grown in RPMI 1640/B27 containing 100 ng/ml Activin A and cells were changed daily. Second, for hepatic specification, cells were then cultured during 2 days in RPMI/B27 supplemented with 10 ng/ml fibroblast growth factor (FGF) 10 (Miltenyi Biotec, Paris, France) and 10 ng/ml bone morphogenetic protein (BMP) 4 (R&D systems, Minneapolis, MN, USA) with daily medium change. Cells were then split using TrypLE in 3 plates precoated with 5 µg/ml laminin 111 or 2 mg/ml matrigel at a cell density of 70×10³ cells/cm². They were cultured in RPMI/B27 supplemented with 10 ng/ml FGF 10, 10 ng/ml BMP-4 and 10 µM rock inhibitor for 1 day and rock inhibitor was omitted from the medium the following day. Finally, for hepatic maturation, cells were cultured in hepatocyte culture medium (Lonza, Bâle, Suisse) supplemented with 20 ng/mL hepatocyte growth factor (HGF) (Miltenyi Biotec, Paris, France) and 20 ng/mL oncostatinM (OSM) (Miltenyi Biotec, Paris, France) (maturation medium) for 4 days. The following days, HGF was omitted from the culture medium and the medium was changed every 2 days.

**Immunofluorescence Assay:** Cultured cells were fixed with 4% paraformaldehyde for 20 min at room temperature, permeabilized with 0.5% Triton X-100 in PBS for 15 min and blocked with 3% BSA in PBS for 15 min. The cells were incubated with primary antibodies for one hour at room temperature. The primary antibodies against human AAT (1:100), CK19 (1:50), and AFP (1:300) were purchased from DAKO (DakoCytomation, Trappes, France); antibodies against human Oct4 (1:100), HNF4α (1:100) were purchased from TebuBio (Le Perray-en-Yvelines, France).. After several washes with PBS 0.1% Triton, with anti-mouse, anti-goat (both from Life Technologies) or anti-rabbit (Abcam) IgG secondary antibodies, conjugated with ALEXA 488, 555 or 647, diluted 1/1000 in PBS 1% BSA 0.1% Triton, for 30 minutes at RT. Cells were then counterstained with DAPI, diluted 1/10,000 in PBS, for 1 minute at RT, and read on an inverted fluorescence microscope (EvosFL from AMG).To evaluate the proportion of differentiated cells, random pictures from 10 independent differentiation were taken, then positive cells for differentiation markers were counted together with whole cells.

**Flow Cytometry:** For flow cytometry, differentiated cells were incubated 2 min with Accutase at 37°C. Dissociated cells were fixed with 0.25% paraformaldehyde for 30 min at 4°C, then permeabilized with 0.2% Tween-20 in PBS for 15 min at 37°C. Cells were blocked with 3% BSA/PBS for 30min/4°C in the presence or absence of primary antibodies diluted in 0.5% BSA in PBS. After washing (with PBS) cells were incubated with 3% BSA/PBS following by a 20 min incubation at 4°C with a donkey anti-mouse Alexa Fluor 488 conjugated antibody. Flow cytometry analysis was performed using a BD LSR II Flow Cytometer (BD Biosciences).

**Animal studies:** Animals were housed in the animal facilities of Nantes University Medical School (Nantes, France) and were maintained under a 12-hour light cycle, fed *ad libitum,* receiving human care according to the guidelines of the French Ministère de l'Agriculture. Homozygous (j/j) Gunn rats weighing 120±30 g (age: 8-9 week-old) were used for the study.

**Liver function tests:** Blood was drawn from retro-orbital sinus. Serum total bilirubin and alanine and aspartate aminotransferases were measured at the routine biochemistry department of Nantes University hospital.

**Histology and Immunohistochemistry:** Immunohistochemical analysis involved the use of immunoperoxidase techniques on fixed paraffin embedded sections. Rabbit polyclonal anti-UGT1A1 (1:50; Abgent Inc., San Diego, USA), anti-alpha feto-protein (1:250; Dako, Glostrup, Denmark), and mouse monoclonal anti-serum albumin (1:200; R&D Systems Europe; Abingdon, UK) antibodies were used for human cell detection. Briefly, 4 µm-thick liver sections were dewaxed and pretreated in citrate buffer (pH=6, 10%, Dako) for 40 min at 98°C. After a treatment in H2O2 (Dako) for 5 min and in TBS Tween (ScyTek Laboratories, West Logan, USA) for another 5 min, sections were incubated with the primary antibody with BSA 2% and TBS Tween for 30 min at 37°C. Bound antibodies were detected using Envision secondary agent (Dako) and DAB Liquid Substrate (Dako) for immunoperoxidase.

**RT-qPCR:** Total mRNA was isolated from cultured cells using the RNeasy minikit (QIAGEN) according to the manufacturer's specifications or TRIzol^{®} (Lifetechnology, Carlsbad, CA), following the manufacturer's instructions. Isolated mRNA was quantified using a Nanodrop, and a total amount of 10ng was used per reaction. Analyses of transcripts were performed with a ViiA7 sequence detection system (Life Technology, Carlsbad, CA, USA) using Power EXPRESS One-Step SYBR^{®}GreenER^{™} Kit, (Life Technology, Carlsbad, CA, USA). Primers sequences are as follows:

| Name | Sequence | SEQ ID NO: |
|---|---|---|
| AFP-F | GCT TGG TGG TGG ATG AAA CA | 1 |
| AFP-R | TCC TCT GTT ATT TGT GGC TTT TG | 2 |
| ALB-F | GCA CAG AAT CCT TGG TGA ACA G | 3 |
| ALB-R | ATG GAA GGT GAA TGT TTT CAG CA | 4 |
| CK 19-F | CTC CCG CGA CTA CAG CCA CT | 5 |
| CK19-R | TCA GCT CAT CCA GCA CCC TG | 6 |
| CYP3A4-F | AGATGCCTTTAGGTCCAATGGG | 7 |
| CYP3A4-R | GCTGGAGATAGCAATGTTCGT | 8 |
| CYP3A7-F | AAGGTCGCCTCAAAGAGACA | 9 |
| CYP3A7-R | TGCACTTTCTGCTGGACATC | 10 |
| FOXA2-F | GCACTCGGCTTCCAGTATG | 11 |
| FOXA2-R | CACGTACGACGACATGTTCA | 12 |
| GAPDH-F | AAT CCC ATC ACC ATC TTC CA | 13 |
| GAPDH-R | TGG ACT CCA CGA CGT ACT CA | 14 |
| HNF4-F | TGG ACA AAG ACA AGA GGA ACC | 15 |
| HNF4-R | ATA GCT TGA CCT TCG AGT GC | 16 |
| SOX2-F | CCTACTCGCAGCAGGGCACC | 17 |
| SOX2-R | CTCGGCGCCGGGGAGATACA | 18 |
| SOX17-F | TTTCATGGTGTGGGCTAAGG | 19 |
| SOX17-R | CGGCCGGTACTTGTAGTTG | 20 |

The PCR method and the analysis of relative gene expression data using the 2⁻ΔΔ^{Ct} quantification method, after normalization to GAPDH values, was performed as previously described. The mRNA expression level is defined as the fold change in mRNA levels in a given sample relative to levels in undifferentiated cells. The mRNA expression level is calculated as follows: mRNA expression level = 2⁻ΔΔ^{Ct} where ΔΔCt = (Ct_{Target} - Ct_{GAPDH})ₛₐₘₚₗₑ - (Ct_{Target} - Ct_{GAPDH}). Specific amplifications were checked by amplicon melting curves.

Other analyses of transcripts were performed with a ViiA7 sequence detection system (Life Technology, Carlsbad, CA, USA) using AgPath-ID^{™} One-Step RT-PCR Reagents, (Life Technology, Carlsbad, CA, USA), and appropriate primer pair (Applied Biosystems).

| GENE | SUPPLIER | REFERENCE |
|---|---|---|
| OCT4 (POUSF1) | Life Technologies | Hs00999632_g1 |
| NANOG | Life Technologies | Hs04260366_g1 |
| FOXA2 | Life Technologies | Hs00232764_m1 |
| SOX17 | Life Technologies | Hs00751752_s1 |
| HNF4A | Life Technologies | Hs00230853_m1 |
| CK19 (KRT19) | Life Technologies | Hs00761767_s1 |
| AFP | Life Technologies | Hs00173490_m1 |
| CYP3A7 | Life Technologies | Hs00426361_m1 |
| ALB | Life Technologies | Hs00910225_m1 |
| AAT (SERPINA1) | Life Technologies | Hs01097800_m1 |
| GAPDH | Life Technologies | Hs99999905_m1 |

**ELISA:** Secretion of human alpha-fetoprotein (AFP) (Calbiotech) and albumin (Bethyl Laboratories) in the culture supernatant were assessed by ELISA according to the manufacturers instructions.

**Hepatocyte transplantation:** Cells were (1 × 10⁷ cells) were detached from the plates using TrypLE, washed, resuspended in 200 µl physiological serum, and injected with a 26-gauge butterfly needle into the inferior splenic pole of Gunn rats that had been subjected 24 hrs earlier to a two-third partial hepatectomy to create an environment optimal for cell transplantation [48]. Rats were immunosuppressed daily with tacrolimus at 0.2 mg/kg one day before cell transplantation for 3 days and at 0.1 mg/kg thereafter. **Statistical analysis:** Data are expressed as mean values ± SEM. Statistical analysis were made using the GraphPad Prim^{®} 5.04 software for Windows (GraphPad Software, San Diego, CA). Statistical significance was assessed using the Mann-Whitney test for comparisons between groups. A p-value <0.05 was considered statistically significant.

### Results

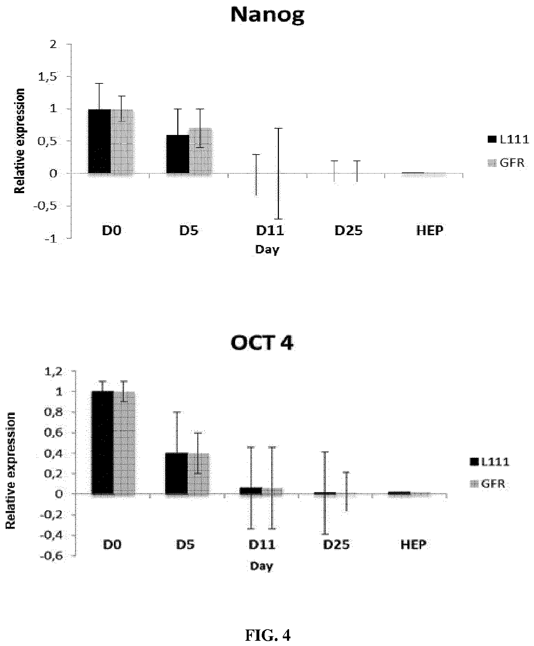

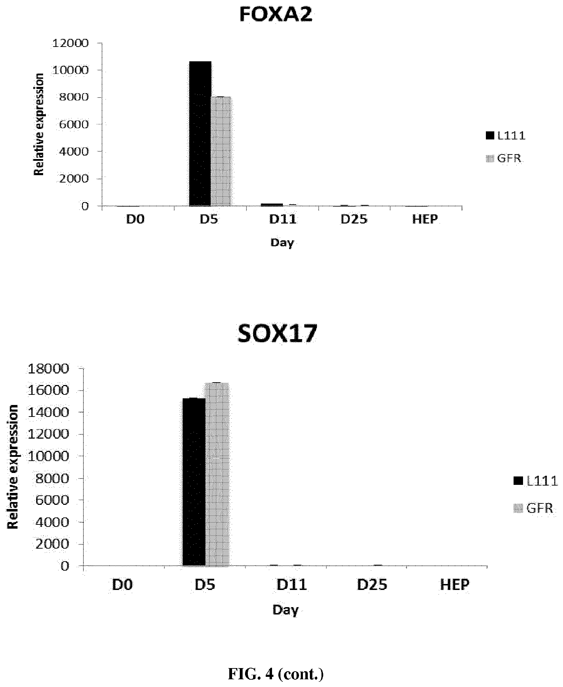

We modified and combined previously reported protocols [16, 20, 36, 40] to develop a GMP-compatible method using chemically-defined conditions xeno-free, feeder-free, and recombinant factors to produced HLCs from hiPSC. Laminin-111 (LN111) is expressed in the fetal liver [41] and the laminin isoform mainly present in Matrigel^{™}. We hypothesized that LN111 would constitute an efficient extracellular matrix for initiating and supporting hepatic differentiation of hiPSC. Our 3-step differentiation protocol to produce HLCs *in vitro* is outlined in **Figure 1A**. We used human iPSC cell line BBHX8, as a representative cell line known to differentiate into HLCs [40, 42]. When hiPSC are at 70-90% confluency, cells were harvested and plated onto dishes coated with recombinant LN111. To obtain more reproducible hepatic differentiation process, cells were enzymatically harvested and single cell suspensions were counted and plated in new LN111-coated dishes, instead of using the standard methods based on empirical cell density visualization to evaluate the time and cell ratio splitting to initiate HLCs production. At day 0, hiPSC were positive for pluripotent markers (Nanog, Sox 2) **(****Figure 1B****)** and were negative for definitive endoderm (Sox 17, FoxA2) (**Figure 1C**) and hepatic markers (HNF4a, ALB, AFP, Cytochrome P450) **(****Figure 1D**). These experiments were confirmed with more accurate RT-qPCR analyses with AgPath-ID^{™} One-Step (**Figure 4**).

Cells were treated with Activin A and basic fibroblast growth factor to generate definitive endoderm. A short-exposure to Wnt3a and rock inhibitor was used to improve expression of definitive endoderm and cell survival after enzymatic cell harvesting, respectively [16, 36]. At day 5, more than 80-90% of the cells strongly expressed the definitive endoderm markers SOX17 and FOXA2 and lost expression of pluripotent genes (**Figure 1B**). The resulting definitive endoderm cells were then cultured in the presence of FGF10 and BMP4, 2 critical factors involved in the initial signaling pathways of early embryonic liver development, as previously described [43]. After 3 days of cell treatment (day 8 of differentiation), cells were enzymatically-passaged on new LN111-coated dishes to expand them and continue hepatic specification stage for 3 days.

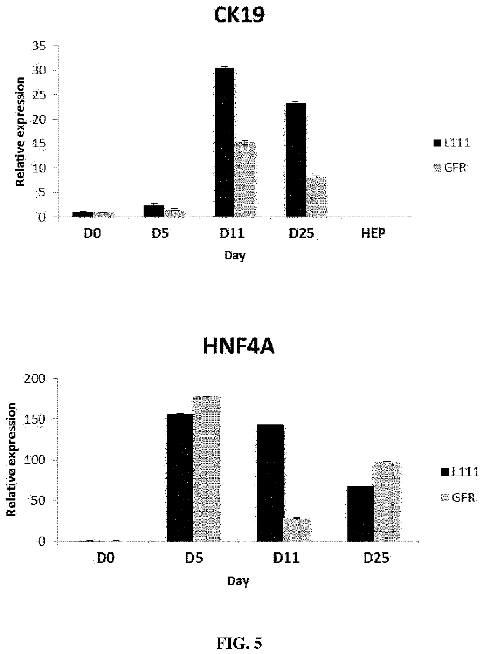

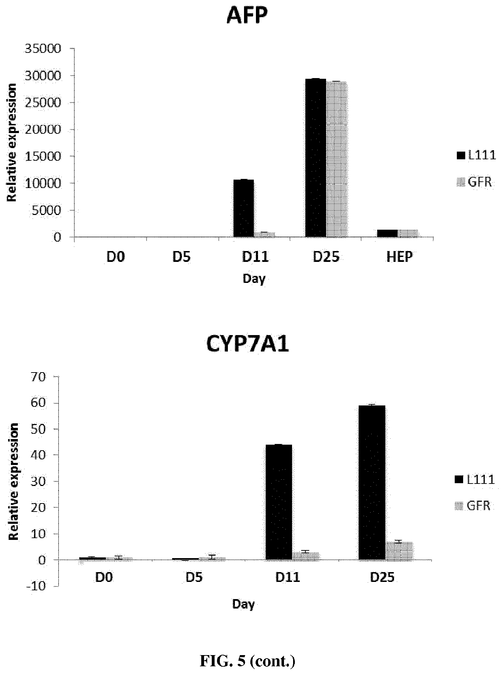

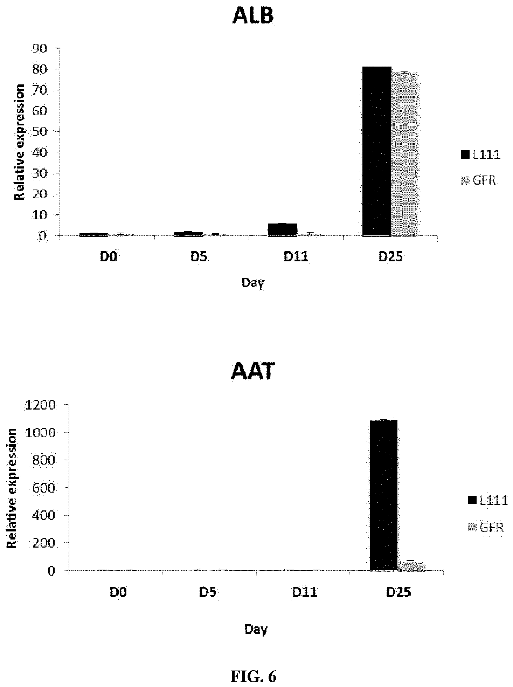

At day 11, almost all cells are positive for markers expressed in hepatic progenitors during the early stages of liver development (HNF4a, CK19 and CYP3A7) **(****Figure 1D**). They were negative for alpha-fetoprotein (AFP), a marker of hepatoblasts, albumin (ALB), AAT (alpha-1 antitrypsin) and cytochrome P450 3A4 (CYP3A4), which are markers of mature hepatocyte. They did not express SOX17 and had a reduced level of FOXA2. To direct differentiation into hepatocyte-like cells (expression of AFP), cells were cultured in the presence of OSM and HGF for 4 days. At the same stage, the cells were tested with the AgPath-ID^{™} One-Step and similar results were obtained (see **Figures 5** and **6**). Altogether, the results showed that they have a phenotype of hepatoblasts [44, 45].

After day 20, hiPSC-derived hepatoblasts expressed HNF4a, CK19, AFP and CYP3A7 (a CYP450 enzyme expressed in fetal hepatocytes) and acquired expression of some markers of mature hepatocytes (ALB, AAT, UGT1A1, CYP3A4) and had lost FOXA2 expression, as assessed by RT-qPCR analyses (**Figure 1D**). In accordant to those results, secretion of AFP in cell supernatant was confirmed by ELISA assays. However, we did not detect secretion of albumin, probably because of the low levels of albumin mRNA expression (**Figure 1E**). Similar results were obtained when hiPSCs were differentiated on matrigel-coated dishes.

Taken together, the results showed that LN111 is as efficient as matrigel for initiating and supporting hepatic differentiation of hiPSC in our culture conditions, but with a recombinant matrix. Our study extended a recent study showing that LN111-coated dishes allowed maintaining hiPSC at a stage of hepatoblast-like cells after hiPSC-endoderm and hepatic specification on matrigel-coated dish [36]. It is in accordance with previous studies showing that HLCs poorly matured *in vitro.* At day 20-30, our HLCs did not secrete albumin, probably because we did not perform culture in the presence of dexamethasone or DMSO that increase HLCs maturity. Highly mature HLCs resemble human primary hepatocytes can be produced if they were culture in 3D culture or micropatterned co-culture conditions [34, 46].

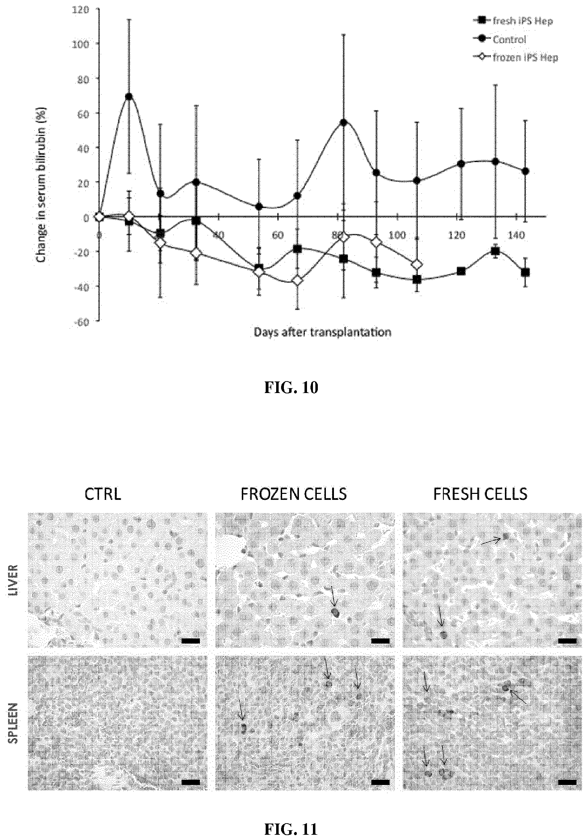

To investigate the therapeutic efficacy, serum bilirubin levels were monitored over time after transplantation of IDHBs (day 11 of hepatic differentiation onto LN111-coated dishes) in immunosuppressed Gunn rats. As shown in **Figure 2**, there was a progressive reduction of baseline bilirubin levels in the treated group following cell transplantation over the course of 60 days. Thereafter, the decrease in serum bilirubinemia persisted at a level of 28 ± 5% throughout the study and was statistically significant compared to pre-transplantation values (P<0.05). Serum bilirubin in the treated group was 57 ± 5 µM at time of sacrifice versus 84 ± 11 µM for pre-transplantation values (p=0.005). Similar decrease in bilirubinemia was observed after transplantation of frozen IDHBs (**Figure 10**). In contrast to treated rats, serum bilirubin in the control group did not decrease and remained elevated with values up to 150 µM. These results were confirmed in for both fresh and frozen hepatocytes (**Figure 10**) Immunochemistry analyses revealed the presence of cells expressing UDP-glucuronosyltransferase 1-A (UGT1A1) confirming that transplanted IDHBs had the capacity to engraft into the liver parenchyma, albeit at a very low level (<0.1%) (see arrows in **Figure 11**). They were distributed throughout the liver parenchyma and mainly as single cells. This is in agreement with the low engraftment efficacy of normal hepatocytes in the liver of animals in which, there is no selective growth advantages of transplanted cells [37, 47-50]. Most of UGT1A1-positive human cells were also detected in the spleen and represented about 1-2% of the spleen (**Figure 11**). No human AFP expressing cells were detected. These data showed that the spleen promoted further maturation of IDHBs into adult hepatocytes. Histology of the liver, as well as of other organs (not shown), of all animals was normal. Serum levels aspartate aminotransferase (AST) and alanine aminotransferase (ALT) (established markers of hepatocyte injury) in treated rats were 1.59 ± 0.21 µkat/l and 1.07± 0.16 µkat/l at day 10 post-transplantation, respectively and 2.07 ± 0.85 µkat/l and 0.95 ± 0.19 µkat/l at time of sacrifice, respectively. These values were not different from pre-operative values (AST:2.44 ± 1 µkat/l and ALT:1.46 ± 0.75 µkat/l) or control rats values at day 10 post-transplantation (AST:1.59 ± 0.12 µkat/l and ALT:1.03 ± 0.12 µkat/l) and at time of sacrifice (AST:1.61 ± 0.53 µkat/l and ALT:1.13 ± 0.33 µkat/l). Gamma-glutamyltransferase values were also normal (<0.05 µkat/l) at these time points. These results showed that animal recovered well from surgical procedures. To follow *in vivo* functionality of transplanted human cells, we measured human AFP and human albumin in animal sera over time. In one animal we could detect significant levels of serum human AFP (above background of control rats serum) at day 11 post-cell transplantation (16 ng/ml) and that progressively became undetectable thereafter. In two other animals, we could detect appearance of serum human albumin after 5 months post-transplantation, albeit at a very low level (6 and 23 pg/ml). No detectable level of human albumin and human AFP was detected in the sera of other engrafted Gunn rats, probably because of the very low number of human cells in the liver.

In summary, we demonstrated for the first time the efficacy of hiPSC-based regenerative for treating an inherited hepatic metabolic disease in absence of any selective growth advantage of transplanted cells over resident rodent hepatocytes, which is the situation encountered in hepatocyte transplantation in humans [9]. After cell transplantation in icteric adult Gunn rats, we demonstrated long-lasting significant decrease in hyperbilirubinemia with freshly prepared and frozen therapeutic human cells. The therapeutic efficacy was equivalent to that obtained with *ex vivo* gene therapy in the Gunn rat using lentivirally-corrected primary hepatocytes [51]. Noteworthy, it was as efficient as transplantation of hepatocytes isolated from neonate human livers and higher than that of hepatocytes isolated from adult human livers, which are the gold standard cells for human cell therapy for treating the Gun rat [61]. In the present study, hiPSCs were differentiated into hepatoblast-like cells that did not express UDP-glucuronosyltransferase 1-A (UGT1A1) for cell transplantation. Our results showed unequivocal evidence that *in situ* maturation have occurred to gain UGT1A1 activity, which was maximal at 8 weeks post-transplantation. We could also detect in some animals, the transient expression of serum AFP and appearance of serum albumin production. Our results are in accordance with previous studies showing that the liver provide for terminal differentiation of immature hepatocytes, i.e. hepatocytes isolated from fetal liver [52, 53]. Recently, it has been reported that hiPSC specified into differentiated hepatoblasts (expressing AFP) were capable to further differentiate in the murine liver, where they lost AFP expression [14, 23, 54, 62]. Our study indicates now that hiPSC specified into hepatoblasts were capable of engrafting the liver of another species and acquired *in situ* maturity at a sufficient level to ensure metabolic hepatic functions in the context of a normal liver regeneration. Furthermore, we showed that the spleen also constitute an adequate site for promoting further differentiation into adult hepatocytes, which was not reported in previous studies with HLCs.

Importantly, *in vivo* functionality of liver engraftment by IDHBs was sustained for a long period (6 months) without signs of tumor formation (carnicoma or teratoma) and liver injury as assessed by histological and blood parameters analyses. Like normal primary hepatocyte transplanted in adult livers [51], IDHBs repopulated the liver as single cells, suggesting that they did respond to anti-proliferate stimuli when liver regeneration have terminated and the liver have returned to mitotic quiescence. In addition, no teratoma was observed 2 months after direct injection of IDHBs in the testis, confirming the absence of residual undifferentiated hiPSC in the HLC cell preparation.

Finally, our study also provide a crucial step towards the clinical application of hiPSC for treating inherited liver diseases, since we have produced the HLCs sing a serum-free, xeno-free and chemically-defined defined hepatic differentiation protocol that fulfills GMP production criteria. We choose to harvest HLCs at a stage of hepatic progenitors (expression of HNF4-alpha and some expression of AFP) because hepatic progenitors isolated from human fetal livers have the ability to engraft and to migrate within the transplanted recipient liver more efficiently than adult hepatocytes [55-57]. It has also been shown that hiPSC-derived endoderm cells have higher engraftment ability than hepatic progenitors-like cells expressing AFP and maturing hepatocytes (expressing albumin) [31]. Conversely, we did not transplant endoderm like-cells (day 5 of differentiation) to reduce the risk of residual undifferentiated hiPSC or not fully differentiated cells in final cell preparations. Loh *et al.* observed that hiPSC-derived hepatic progenitors failed to engraft in murine neonate livers. This discrepancy to the above mentioned studies and ours may be related either to a different hepatic differentiation protocol leading to different engraftment ability of produced HLCs (for instance, Loh *et al.* used several small molecule inhibitors), to difference in the liver recipient (neonate versus adult livers). Besides, in our study, few IDHBs were detected in the liver but this may due to the fact the spleen retained all IDHBs at the time of intrasplenic cell injection ; such intrasplenic cell retention was not described in others studies [60-62].

Harvesting at day 11 of hepatic differentiation (end of hepatic specification step) also simplified and reduced culture time and will facilitate standardization, reproducibility and reduce cost of mass cell production.

In conclusion, there is a clinical demand for human hepatocytes for treating inherited liver diseases, including CN-1. Hepatic progenitors derived from hiPSC, or from hESC, cultured on laminin-coated plates could be a safe clinical alternative to liver transplants and human hepatocytes isolated from cadaveric livers. Although the study was focused on therapies for CN-1, it also provided a significant advance in treatment of other inherited liver diseases, such as urea cycle diseases. Indeed, these diseases could be treated by allogeneic hepatocyte transplantation [9] and thus directly amenable to regenerative medicine approach using normal human pluripotent stem cells. As access to HLCs will not be limited, hepatocyte transplantation could be repeated to maintain or to achieve satisfactory therapeutic benefits.

### EXAMPLE 2: USE OF LN-521 AS A MATRIX FOR DIFFERENTIATING PLURIPOTENT CELLS INTO HEPATOCYTE LINEAGE CELLS.

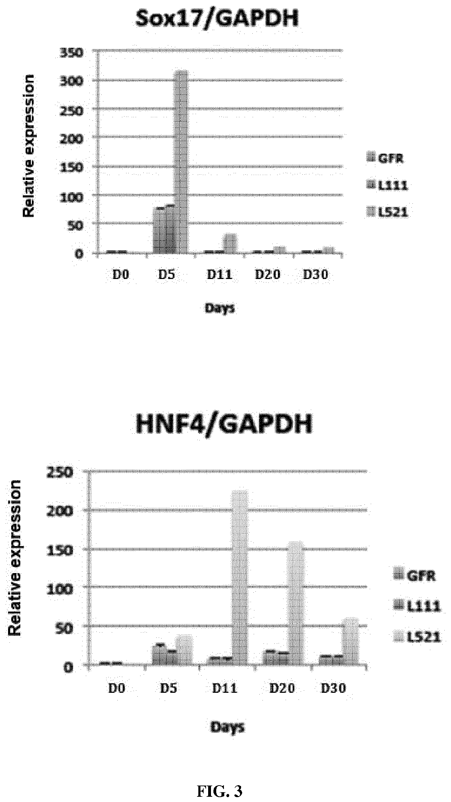

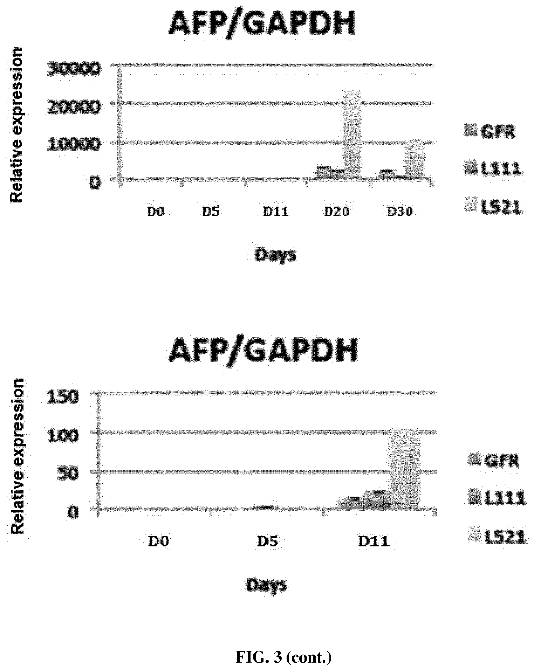

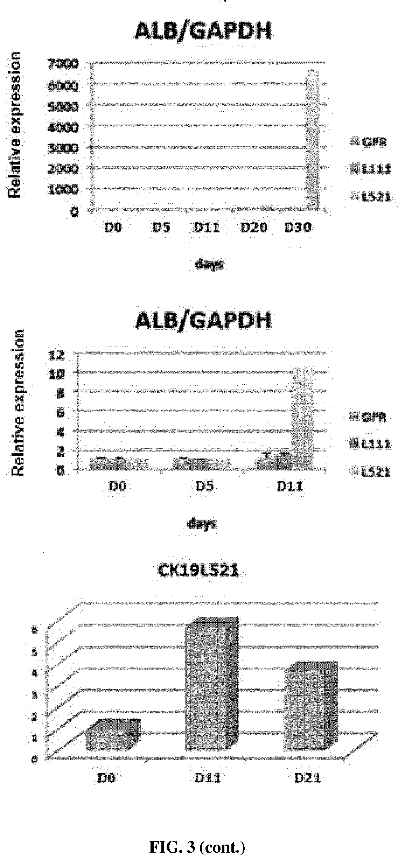

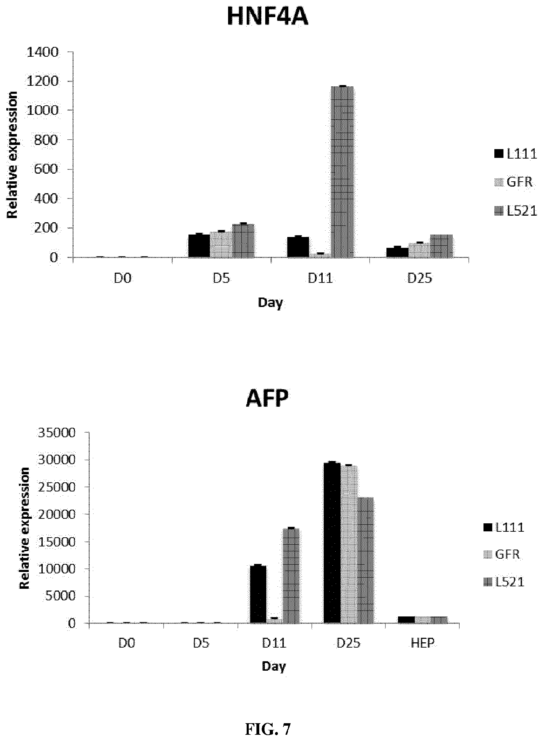

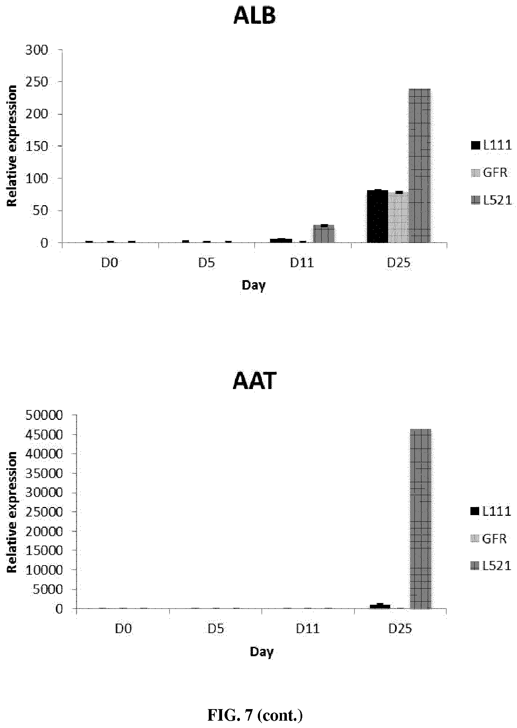

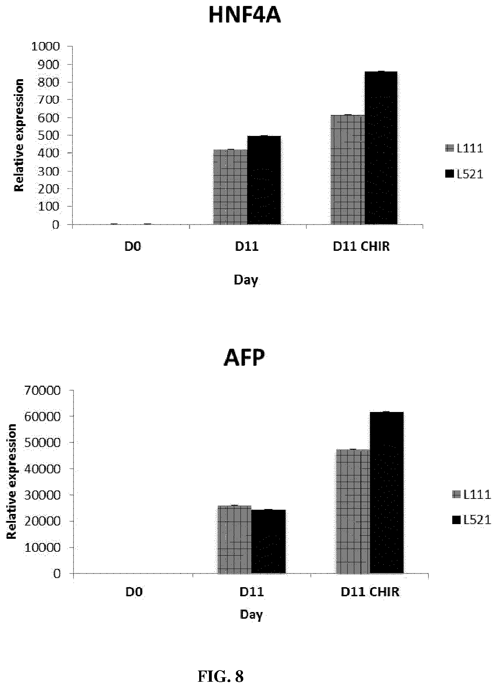

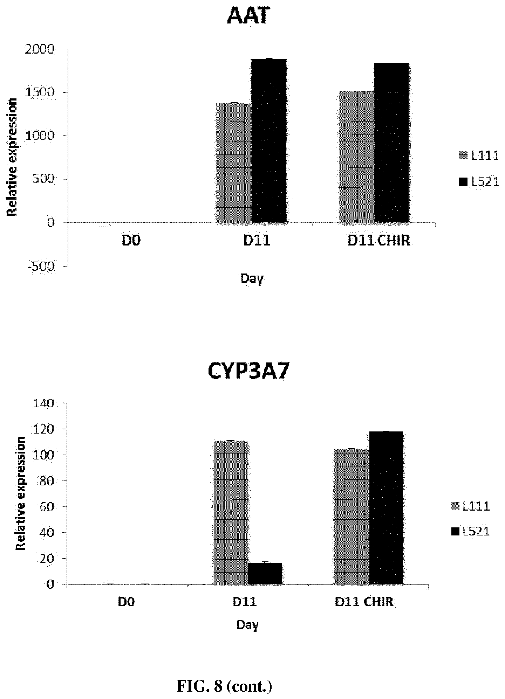

Interestingly, recombinant human laminin-521 (LN-521) can substitute LN-111 as extracellular matrix and allowed better differentiation of hiPSC into definitive endoderm and HLCs derived from hiPSCs as assessed by RT-qPCR analyses of HNF4alpha, AFP, Albumin, AAT expression gene expression. For instance, we observed higher expression of SOX17 (endoderm) at day 5 of differentiation, higher expression of HNF4alpha, AFP and albumin from day 11 to day 30, as assessed by sybregreen based-RT-qPCR (**Figure** 3). These results were confirmed using Taqman-based RT-qPCR for HLCs derived from hiPSC (**Figure 7**) and HLCs derived from hESCs (ESI-017 cell line) (**Figure 8**).

When CHIR 99021 was added for 24h at day 0 of the hepatic differentiation process, the difference between LN-521 and LN-111 is more pronounced (**Figure 8**).

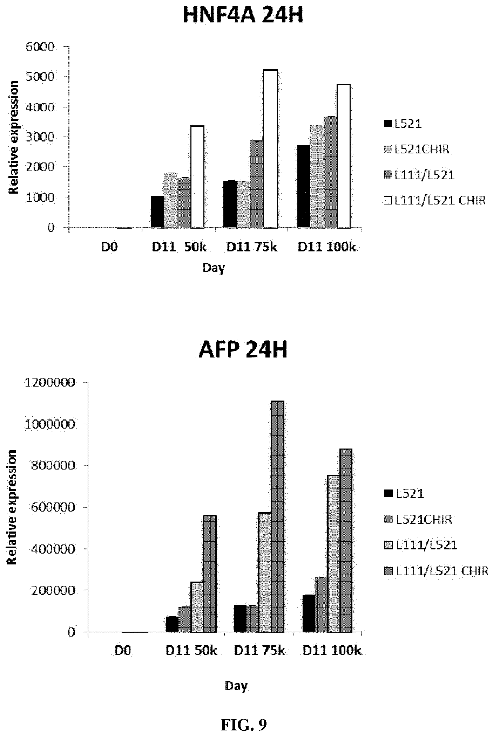

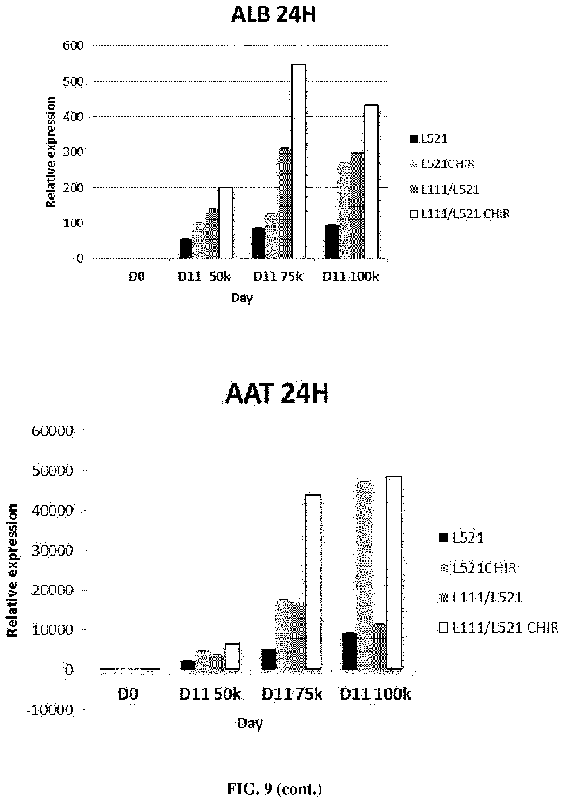

Interestingly, when we used a mix of LN-521 and LN-111 (25%/75%), hepatic differentiation of pluripotent stem cells (hESCs) is more efficient as compared to LN-521 or LN-111 alone (**Figure 9**). This improvement was observed at different cell seeding (50×10³, 75×10³ and 100×10³ cells). Again, we observed that adding CHIR 99021 improved the efficacy of hepatic differentiation.

### REFERENCES

[1] K. Takahashi, K. Tanabe, M. Ohnuki, M. Narita, T. Ichisaka, K. Tomoda, S. Yamanaka, Induction of pluripotent stem cells from adult human fibroblasts by defined factors, Cell, 131 (2007) 861-872.
[2] J. Yu, M.A. Vodyanik, K. Smuga-Otto, J. Antosiewicz-Bourget, J.L. Frane, S. Tian, J. Nie, G.A. Jonsdottir, V. Ruotti, R. Stewart, Slukvin, II, J.A. Thomson, Induced pluripotent stem cell lines derived from human somatic cells, Science, 318 (2007) 1917-1920.
[3] P. Menasche, V. Vanneaux, J.R. Fabreguettes, A. Bel, L. Tosca, S. Garcia, V. Bellamy, Y. Farouz, J. Pouly, O. Damour, M.C. Perier, M. Desnos, A. Hagege, O. Agbulut, P. Bruneval, G. Tachdjian, J.H. Trouvin, J. Larghero, Towards a clinical use of human embryonic stem cell-derived cardiac progenitors: a translational experience, European heart journal, (2014).
[4] S. Nedelec, B. Onteniente, M. Peschanski, C. Martinat, Genetically-modified human pluripotent stem cells: new hopes for the understanding and the treatment of neurological diseases?, Curr Gene Ther, 13 (2013) 111-119.
[5] N. Dianat, C. Steichen, L. Vallier, A. Weber, A. Dubart-Kupperschmitt, Human pluripotent stem cells for modelling human liver diseases and cell therapy, Curr Gene Ther, 13 (2013) 120-132.
[6] S.D. Schwartz, C.D. Regillo, B.L. Lam, D. Eliott, P.J. Rosenfeld, N.Z. Gregori, J.P. Hubschman, J.L. Davis, G. Heilwell, M. Spirn, J. Maguire, R. Gay, J. Bateman, R.M. Ostrick, D. Morris, M. Vincent, E. Anglade, L.V. Del Priore, R. Lanza, Human embryonic stem cell-derived retinal pigment epithelium in patients with age-related macular degeneration and Stargardt's macular dystrophy: follow-up of two open-label phase 1/2 studies, Lancet, (2014).
[7] F.W. Pagliuca, J.R. Millman, M. Gurtler, M. Segel, A. Van Dervort, J.H. Ryu, Q.P. Peterson, D. Greiner, D.A. Melton, Generation of functional human pancreatic beta cells in vitro, Cell, 159 (2014) 428-439.
[8] A. Rezania, J.E. Bruin, P. Arora, A. Rubin, I. Batushansky, A. Asadi, S. O'Dwyer, N. Quiskamp, M. Mojibian, T. Albrecht, Y.H. Yang, J.D. Johnson, T.J. Kieffer, Reversal of diabetes with insulin-producing cells derived in vitro from human pluripotent stem cells, Nat Biotechnol, 32 (2014) 1121-1133.
[9] A. Dhawan, J. Puppi, R.D. Hughes, R.R. Mitry, Human hepatocyte transplantation: current experience and future challenges, Nat Rev Gastroenterol Hepatol, 7 (2010) 288-298.
[10] J.H. Waelzlein, J. Puppi, A. Dhawan, Hepatocyte transplantation for correction of inborn errors of metabolism, Current opinion in nephrology and hypertension, 18 (2009) 481-488.
[11] I.J. Fox, J.R. Chowdhury, S.S. Kaufman, T.C. Goertzen, N.R. Chowdhury, P.I. Warkentin, K. Dorko, B.V. Sauter, S.C. Strom, Treatment of the Crigler-Najjar syndrome type I with hepatocyte transplantation, N Engl J Med, 338 (1998) 1422-1426.
[12] C. Ribes-Koninckx, E.P. Ibars, M.A. Agrasot, A. Bonora-Centelles, B.P. Miquel, J.J. Vila Carbo, E.D. Aliaga, J.M. Pallardo, M.J. Gomez-Lechon, J.V. Castell, Clinical outcome of hepatocyte transplantation in four pediatric patients with inherited metabolic diseases, Cell Transplant, 21 (2012) 2267-2282.
[13] K. Takayama, Y. Morisaki, S. Kuno, Y. Nagamoto, K. Harada, N. Furukawa, M. Ohtaka, K. Nishimura, K. Imagawa, F. Sakurai, M. Tachibana, R. Sumazaki, E. Noguchi, M. Nakanishi, K. Hirata, K. Kawabata, H. Mizuguchi, Prediction of interindividual differences in hepatic functions and drug sensitivity by using human iPS-derived hepatocytes, Proc Natl Acad Sci U S A, 111 (2014) 16772-16777.
[14] A. Carpentier, A. Tesfaye, V. Chu, I. Nimgaonkar, F. Zhang, S.B. Lee, S.S. Thorgeirsson, S.M. Feinstone, T.J. Liang, Engrafted human stem cell-derived hepatocytes establish an infectious HCV murine model, J Clin Invest, 124 (2014) 4953-4964.
[15] S. Gerbal-Chaloin, N. Funakoshi, A. Caillaud, C. Gondeau, B. Champon, K. Si-Tayeb, Human Induced Pluripotent Stem Cells in Hepatology: Beyond the Proof of Concept, Am J Pathol, 184 (2014) 332-347.
[16] M. Kajiwara, T. Aoi, K. Okita, R. Takahashi, H. Inoue, N. Takayama, H. Endo, K. Eto, J. Toguchida, S. Uemoto, S. Yamanaka, Donor-dependent variations in hepatic differentiation from human-induced pluripotent stem cells, Proc Natl Acad Sci USA, 109 (2012) 12538-12543.
[17] H. Basma, A. Soto-Gutierrez, G.R. Yannam, L. Liu, R. Ito, T. Yamamoto, E. Ellis, S.D. Carson, S. Sato, Y. Chen, D. Muirhead, N. Navarro-Alvarez, R.J. Wong, J. Roy-Chowdhury, J.L. Platt, D.F. Mercer, J.D. Miller, S.C. Strom, N. Kobayashi, I.J. Fox, Differentiation and transplantation of human embryonic stem cell-derived hepatocytes, Gastroenterology, 136 (2009) 990-999.
[18] J. Cai, Y. Zhao, Y. Liu, F. Ye, Z. Song, H. Qin, S. Meng, Y. Chen, R. Zhou, X. Song, Y. Guo, M. Ding, H. Deng, Directed differentiation of human embryonic stem cells into functional hepatic cells, Hepatology, 45 (2007) 1229-1239.
[19] D.C. Hay, J. Fletcher, C. Payne, J.D. Terrace, R.C. Gallagher, J. Snoeys, J.R. Black, D. Wojtacha, K. Samuel, Z. Hannoun, A. Pryde, C. Filippi, I.S. Currie, S.J. Forbes, J.A. Ross, P.N. Newsome, J.P. Iredale, Highly efficient differentiation of hESCs to functional hepatic endoderm requires ActivinA and Wnt3a signaling, Proc Natl Acad Sci USA, 105 (2008) 12301-12306.
[20] K. Si-Tayeb, F.K. Noto, M. Nagaoka, J. Li, M.A. Battle, C. Duris, P.E. North, S. Dalton, S.A. Duncan, Highly efficient generation of human hepatocyte-like cells from induced pluripotent stem cells, Hepatology, 51 (2010) 297-305.
[21] G.J. Sullivan, D.C. Hay, I.H. Park, J. Fletcher, Z. Hannoun, C.M. Payne, D. Dalgetty, J.R. Black, J.A. Ross, K. Samuel, G. Wang, G.Q. Daley, J.H. Lee, G.M. Church, S.J. Forbes, J.P. Iredale, I. Wilmut, Generation of functional human hepatic endoderm from human induced pluripotent stem cells, Hepatology, 51 (2010) 329-335.
[22] M. Baxter, S. Withey, S. Harrison, C.P. Segeritz, F. Zhang, R. Atkinson-Dell, C. Rowe, D.T. Gerrard, R. Sison-Young, R. Jenkins, J. Henry, A.A. Berry, L. Mohamet, M. Best, S.W. Fenwick, H. Malik, N.R. Kitteringham, C.E. Goldring, K.P. Hanley, L. Vallier, N.A. Hanley, Phenotypic and functional analyses show stem cell-derived hepatocyte-like cells better mimic fetal rather than adult hepatocytes, J Hepatol, (2014).
[23] K.M. Loh, L.T. Ang, J. Zhang, V. Kumar, J. Ang, J.Q. Auyeong, K.L. Lee, S.H. Choo, C.Y. Lim, M. Nichane, J. Tan, M.S. Noghabi, L. Azzola, E.S. Ng, J. Durruthy-Durruthy, V. Sebastiano, L. Poellinger, A.G. Elefanty, E.G. Stanley, Q. Chen, S. Prabhakar, I.L. Weissman, B. Lim, Efficient endoderm induction from human pluripotent stem cells by logically directing signals controlling lineage bifurcations, Cell stem cell, 14 (2014) 237-252.
[24] K. Yusa, S.T. Rashid, H. Strick-Marchand, I. Varela, P.Q. Liu, D.E. Paschon, E. Miranda, A. Ordonez, N.R. Hannan, F.J. Rouhani, S. Darche, G. Alexander, S.J. Marciniak, N. Fusaki, M. Hasegawa, M.C. Holmes, J.P. Di Santo, D.A. Lomas, A. Bradley, L. Vallier, Targeted gene correction of alpha1-antitrypsin deficiency in induced pluripotent stem cells, Nature, 478 (2011) 391-394.
[25] S.E. Kim, S.Y. An, D.H. Woo, J. Han, J.H. Kim, Y.J. Jang, J.S. Son, H. Yang, Y.P. Cheon, J.H. Kim, Engraftment potential of spheroid-forming hepatic endoderm derived from human embryonic stem cells, Stem cells and development, 22 (2013) 1818-1829.
[26] S. Asgari, M. Moslem, K. Bagheri-Lankarani, B. Pournasr, M. Miryounesi, H. Baharvand, Differentiation and transplantation of human induced pluripotent stem cell-derived hepatocyte-like cells, Stem cell reviews, 9 (2013) 493-504.
[27] M. Vosough, E. Omidinia, M. Kadivar, M.A. Shokrgozar, B. Pournasr, N. Aghdami, H. Baharvand, Generation of Functional Hepatocyte-Like Cells from Human Pluripotent Stem Cells in a Scalable Suspension Culture, Stem cells and development, 22 (2013) 2693-2705.
[28] T. Takebe, K. Sekine, M. Enomura, H. Koike, M. Kimura, T. Ogaeri, R.R. Zhang, Y. Ueno, Y.W. Zheng, N. Koike, S. Aoyama, Y. Adachi, H. Taniguchi, Vascularized and functional human liver from an iPSC-derived organ bud transplant, Nature, 499 (2013) 481-484.
[29] Y.F. Chen, C.Y. Tseng, H.W. Wang, H.C. Kuo, V.W. Yang, O.K. Lee, Rapid generation of mature hepatocyte-like cells from human induced pluripotent stem cells by an efficient three-step protocol, Hepatology, 55 (2012) 1193-1203.
[30] H.Y. Li, Y. Chien, Y.J. Chen, S.F. Chen, Y.L. Chang, C.H. Chiang, S.Y. Jeng, C.M. Chang, M.L. Wang, L.K. Chen, S.I. Hung, T.I. Huo, S.D. Lee, S.H. Chiou, Reprogramming induced pluripotent stem cells in the absence of c-Myc for differentiation into hepatocyte-like cells, Biomaterials, 32 (2011) 5994-6005.
[31] H. Liu, Y. Kim, S. Sharkis, L. Marchionni, Y.Y. Jang, In vivo liver regeneration potential of human induced pluripotent stem cells from diverse origins, Science translational medicine, 3 (2011) 82ra39.
[32] S. Agarwal, K.L. Holton, R. Lanza, Efficient differentiation of functional hepatocytes from human embryonic stem cells, Stem Cells, 26 (2008) 1117-1127.
[33] A. Soto-Gutierrez, N. Kobayashi, J.D. Rivas-Carrillo, N. Navarro-Alvarez, D. Zhao, T. Okitsu, H. Noguchi, H. Basma, Y. Tabata, Y. Chen, K. Tanaka, M. Narushima, A. Miki, T. Ueda, H.S. Jun, J.W. Yoon, J. Lebkowski, N. Tanaka, I.J. Fox, Reversal of mouse hepatic failure using an implanted liver-assist device containing ES cell-derived hepatocytes, Nat Biotechnol, 24 (2006) 1412-1419.
[34] D.R. Berger, B.R. Ware, M.D. Davidson, S.R. Allsup, S.R. Khetani, Enhancing the functional maturity of iPSC-derived human hepatocytes via controlled presentation of cell-cell interactions in vitro, Hepatology, (2014).
[35] K.G. Chen, B.S. Mallon, R.D. McKay, P.G. Robey, Human pluripotent stem cell culture: considerations for maintenance, expansion, and therapeutics, Cell stem cell, 14 (2014) 13-26.
[36] K. Takayama, Y. Nagamoto, N. Mimura, K. Tashiro, F. Sakurai, M. Tachibana, T. Hayakawa, K. Kawabata, H. Mizuguchi, Long-Term Self-Renewal of Human ES/iPS-Derived Hepatoblast-like Cells on Human Laminin 111-Coated Dishes, Stem cell reports, 1 (2013) 322-335.
[37] J. Birraux, O. Menzel, B. Wildhaber, C. Jond, T.H. Nguyen, C. Chardot, A step toward liver gene therapy: efficient correction of the genetic defect of hepatocytes isolated from a patient with Crigler-Najjar syndrome type 1 with lentiviral vectors, Transplantation, 87 (2009) 1006-1012.
[38] P.A. Lysy, M. Najimi, X. Stephenne, A. Bourgois, F. Smets, E.M. Sokal, Liver cell transplantation for Crigler-Najjar syndrome type I: update and perspectives, World J Gastroenterol, 14 (2008) 3464-3470.
[39] T.H. Nguyen, N. Ferry, Gene therapy for liver enzyme deficiencies: What have we learned from models for Crigler-Najjar and Tyrosinemia, Expert Rev Gastroenterol Hepatol, 1 (2007) 155-177.
[40] N.R. Hannan, C.P. Segeritz, T. Touboul, L. Vallier, Production of hepatocyte-like cells from human pluripotent stem cells, Nature protocols, 8 (2013) 430-437.
[41] P. Ekblom, P. Lonai, J.F. Talts, Expression and biological role of laminin-1, Matrix Biol, 22 (2003) 35-47.
[42] S. Pauklin, L. Vallier, The cell-cycle state of stem cells determines cell fate propensity, Cell, 155 (2013) 135-147.
[43] T. Touboul, N.R. Hannan, S. Corbineau, A. Martinez, C. Martinet, S. Branchereau, S. Mainot, H. Strick-Marchand, R. Pedersen, J. Di Santo, A. Weber, L. Vallier, Generation of functional hepatocytes from human embryonic stem cells under chemically defined conditions that recapitulate liver development, Hepatology, 51 (2010) 1754-1765.
[44] G. Lanzoni, T. Oikawa, Y. Wang, C.B. Cui, G. Carpino, V. Cardinale, D. Gerber, M. Gabriel, J. Dominguez-Bendala, M.E. Furth, E. Gaudio, D. Alvaro, L. Inverardi, L.M. Reid, Concise review: clinical programs of stem cell therapies for liver and pancreas, Stem Cells, 31 (2013) 2047-2060.
[45] S.N. de Wildt, G.L. Keams, J.S. Leeder, J.N. van den Anker, Cytochrome P450 3A: ontogeny and drug disposition, Clinical pharmacokinetics, 37 (1999) 485-505.
[46] R.L. Gieseck Iii, N.R. Hannan, R. Bort, N.A. Hanley, R.A. Drake, G.W. Cameron, T.A. Wynn, L. Vallier, Maturation of Induced Pluripotent Stem Cell Derived Hepatocytes by 3D-Culture, PloS one, 9 (2014) e86372.
[47] T.H. Nguyen, J. Birraux, B. Wildhaber, A. Myara, F. Trivin, C. Le Coultre, D. Trono, C. Chardot, Ex vivo lentivirus transduction and immediate transplantation of uncultured hepatocytes for treating hyperbilirubinemic Gunn rat, Transplantation, 82 (2006) 794-803.
[48] T.H. Nguyen, J. Oberholzer, J. Birraux, P. Majno, P. Morel, D. Trono, Highly efficient lentiviral vector-mediated transduction of nondividing, fully reimplantable primary hepatocytes, Mol Ther, 6 (2002) 199-209.
[49] S. Gupta, P. Rajvanshi, R. Sokhi, S. Slehria, A. Yam, A. Kerr, P.M. Novikoff, Entry and integration of transplanted hepatocytes in rat liver plates occur by disruption of hepatic sinusoidal endothelium, Hepatology, 29 (1999) 509-519.
[50] C. Guha, A. Sharma, S. Gupta, A. Alfieri, G.R. Gorla, S. Gagandeep, R. Sokhi, N. Roy-Chowdhury, K.E. Tanaka, B. Vikram, J. Roy-Chowdhury, Amelioration of radiation-induced liver damage in partially hepatectomized rats by hepatocyte transplantation, Cancer Res, 59 (1999) 5871-5874.
[51] T.H. Nguyen, M. Bellodi-Privato, D. Aubert, V. Pichard, A. Myara, D. Trono, N. Ferry, Therapeutic lentivirus-mediated neonatal in vivo gene therapy in hyperbilirubinemic Gunn rats, Mol Ther, 12 (2005) 852-859.
[52] T. Cantz, D.M. Zuckerman, M.R. Burda, M. Dandri, B. Goricke, S. Thalhammer, W.M. Heckl, M.P. Manns, J. Petersen, M. Ott, Quantitative gene expression analysis reveals transition of fetal liver progenitor cells to mature hepatocytes after transplantation in uPA/RAG-2 mice, Am J Pathol, 162 (2003) 37-45.
[53] J.S. Sandhu, P.M. Petkov, M.D. Dabeva, D.A. Shafritz, Stem cell properties and repopulation of the rat liver by fetal liver epithelial progenitor cells, Am J Pathol, 159 (2001) 1323-1334.
[54] S. Zhu, M. Rezvani, J. Harbell, A.N. Mattis, A.R. Wolfe, L.Z. Benet, H. Willenbring, S. Ding, Mouse liver repopulation with hepatocytes generated from human fibroblasts, Nature, 508 (2014) 93-97.
[55] J.E. Allain, I. Dagher, D. Mahieu-Caputo, N. Loux, M. Andreoletti, K. Westerman, P. Briand, D. Franco, P. Leboulch, A. Weber, Immortalization of a primate bipotent epithelial liver stem cell, Proc Natl Acad Sci U S A, 99 (2002) 3639-3644.
[56] J.P. Delgado, V. Vanneaux, J. Branger, T. Touboul, L. Sentilhes, S. Mainot, P. Lainas, P. Leclerc, G. Uzan, D. Mahieu-Caputo, A. Weber, The role of HGF on invasive properties and repopulation potential of human fetal hepatic progenitor cells, Exp Cell Res, 315 (2009) 3396-3405.
[57] D. Mahieu-Caputo, J.E. Allain, J. Branger, A. Coulomb, J.P. Delgado, M. Andreoletti, S. Mainot, R. Frydman, P. Leboulch, J.P. Di Santo, F. Capron, A. Weber, Repopulation of athymic mouse liver by cryopreserved early human fetal hepatoblasts, Hum Gene Ther, 15 (2004) 1219-1228.
[58] M. Black, B.H. Billing, K.P. Heirwegh, Determination of bilirubin UDP-glucuronyl transferase activity in needle-biopsy specimens of human liver, Clinica chimica acta; international journal of clinical chemistry, 29 (1970) 27-35.
[59] M. Muraca, N. Blanckaert, Liquid-chromatographic assay and identification of mono- and diester conjugates of bilirubin in normal serum, Clin Chem, 29 (1983) 1767-1771.
[60] Chen Y, Li Y, Wang X, Zhang W, Sauer V, Chang CJ, Han B, Tchaikovskaya T, Avsar Y, Tafaleng E, Madhusudana Girija S, Tar K, Polgar Z, Strom S, Bouhassira EE, Guha C, Fox IJ, Roy-Chowdhury J, Roy-Chowdhury N. Amelioration of perbilirubinemia in Gunn Rats after transplantation of Human Induced Pluripotent Stem Cell-Derived Hepatocytes. Stem Cell Reports. (2015) 5(1): 22-30.
[61] Tolosa L, López S, Pareja E, Donato MT, Myara A, Nguyen TH, Castell JV, Gómez-Lechón MJ. Human neonatal hepatocyte transplantation induces long-term rescue of unconjugated hyperbilirubinemia in the Gunn rat. Liver Transpl. 2015 Jun;21(6):801-11.
[62] Engrafted human stem cell-derived hepatocytes establish an infectious HCV murine model. Carpentier A, Tesfaye A, Chu V, Nimgaonkar I, Zhang F, Lee SB, Thorgeirsson SS, Feinstone SM, Liang TJ. J Clin Invest. 2014 Nov; 124(11):4953-64.
[63] Cooper AR et al. J Cell Mol Med 2009; 13:2622e33; Rodin S et al. Nat. Commun, 2014; 5: 3195.
[64] Seebacher T et al. Exp Cell Res. 1997; 237(1):70-6.
[65] I. Virtanen, Det al. Exp. Cell Res., 2000; 257: 298-309.
[66] Long-term self-renewal of human ES/iPS-derived hepatoblast-like cells on human laminin 111-coated dishes - Kazuo Takayama, Yasuhito Nagamoto, Natsumi Mimura, Katsuhisa Tashiro, Fuminori Sakurai, Masashi Tachibana, Takao Hayakawa, Kenji Kawabata, Hiroyuki Mizuguchi - Stem Cell Reports 2013 Oct 3;1(4):322-35.
[67] Efficient Generation of Hepatoblasts From Human ES Cells and iPS Cells by Transient Overexpression of Homeobox Gene HEX - Mitsuru Inamura, Kenji Kawabata, Katsuhisa Tashiro, Fuminori Sakurai, Kazufumi Katayama, Masashi Toyoda, Hidenori Akutsu, Yoshitaka Miyagawa, Hajime Okita, Nobutaka Kiyokawa, Akihiro Umezawa, Takao Hayakawa, Miro K Furue and Hiroyuki Mizuguchi - Molecular therapy, Volume 19, Issue 2, February 2011, Pages 400-407.
[68] Highly efficient generation of human hepatocyte-like cells from induced pluripotent stem cells - Karim Si-Tayeb, Fallon K Noto, Masato Nagaoka, Jixuan Li, Michele A Battle, Christine Duris, Paula E North, Stephen Dalton, Stephen A Duncan - Hepatology 2010 Jan;51(1):297-305.
[69] Takumi Teratani , Hanako Yamamoto, Kazuhiko Aoyagi, Hiroki Sasaki, Akira Asari, Gary Quinn, Hideo Sasaki, Masaaki Terada, Takahiro Ochiya Direct hepatic fate specification from mouse embryonic stem cells Hepatology. 2005 Apr;41(4):836-46.

## Claims

1. A method for inducing human hepatic differentiation comprising the steps of:
(i) culturing a population of human pluripotent or multipotent cells on a support coated with human laminin in an endoderm induction medium to produce a population of human definitive endoderm (DE) cells,
and wherein said method does not comprise the overexpression of homeobox transgene *HEX* in said population of cells.

2. A method for inducing human hepatic differentiation comprising the steps of:
(i) culturing a population of human definitive endoderm (DE) cells on a support coated with human laminin in a hepatic induction medium to produce a population of human hepatoblast-like cells, and
(ii) optionally culturing said population of human hepatoblast-like cells on a support coated with human laminin in a hepatic maturation medium to produce a population of hepatocyte-like cells, preferably human fetal hepatocyte-like cells,
and wherein said method does not comprise the overexpression of homeobox transgene *HEX* in said population of cells.

3. The method for inducing hepatic differentiation according to claim 1 further comprising the steps of:
(ii) culturing said population of human DE cells on a support coated with human laminin in a hepatic induction medium to produce a population of human hepatoblast-like cells, and
(iii) optionally culturing said population of human hepatoblast-like cells on a support coated with human laminin in a hepatic maturation medium to produce a population of hepatocyte-like cells, preferably human fetal hepatocyte-like cells,
and wherein said method does not comprise the overexpression of homeobox transgene *HEX* in said population of cells.

4. The method according to claim **2** or **3,** wherein the hepatic induction medium is a chemically defined medium comprising bone morphogenetic protein 4 (BMP4) and family growth factor (FGF10 or FGF2).

5. The method according to claim **2** or **3,** wherein the hepatic maturation medium is a chemically defined medium comprising hepatic growth factor (HGF) and oncostatin M (OSM).

6. The method according to claim **1** or any one of claims **3** to **5,** wherein the endoderm induction medium is a chemically defined medium comprising at least Activin A and optionally WNT3A.

7. The method according to any one of claims **1** to **6,** wherein the hepatic induction medium and/or the endoderm induction medium further comprises a ROCK inhibitor and/or CHIR99021.

8. The method according to any one of claims **1** to **7,** wherein the human laminin (LN) is selected from the group consisting of laminin-111 (LN-111), laminin-211 (LN-211), laminin-332 (LN-332), laminin-411 (LN-411), laminin-421 (LN-421), laminin-511 (LN-511) and laminin-521 (LN-521).

9. The method according to claim **8,** wherein the human laminin (LN) is selected from the group consisting of human recombinant laminin-111 (LN-111) and human recombinant laminin-521 (LN-521) and a mixture thereof.

## Patentansprüche

1. Verfahren zum Induzieren von humaner hepatischer Differenzierung, umfassend die Schritte:
(i) Züchten einer Population von menschlichen pluripotenten oder multipotenten Zellen auf einem Träger, der mit menschlichem Laminin in einem Entoderminduktions-Medium beschichtet ist, um eine Population von menschlichen definitiven Entoderm-Zellen (DE-Zellen) zu produzieren und wobei das genannte Verfahren keine Überexpression von Homeobox-Transgen *HEX* in der genannten Zellpopulation umfasst.

2. Verfahren zum Induzieren von humaner hepatischer Differenzierung, umfassend die Schritte:
(i) Züchten einer Population von menschlichen definitiven Entoderm-Zellen (DE-Zellen) auf einem Träger, der mit menschlichem Laminin beschichtet ist, in einem hepatischen Induktionsmedium, um eine Population von menschlichen hepatoblast-ähnlichen Zellen zu produzieren, und
(ii) optional Züchten der genannten Population von menschlichen heptaoblast-ähnlichen Zellen auf einem Träger, der mit menschlichem Laminin beschichtet ist, in einem hepatischen Maturationsmedium, um eine Population von hepatozyt-ähnlichen Zellen, bevorzugt menschlichen fötalen hepatozyt-ähnlichen Zellen, zu produzieren,
und wobei das genannte Verfahren die Überexpression des Homeobox-Transgens *HEX* in der genannten Zellpopulation nicht umfasst.

3. Verfahren zum Induzieren einer hepatischen Differenzierung gemäß Anspruch 1, weiterhin umfassend die Schritte:
(ii) Züchten der genannten Population von menschlichen DE-Zellen auf einem Träger, der mit menschlichem Laminin beschichtet ist, in einem hepatischen Induktionsmedium, um eine Population von menschlichen hepatoblast-ähnlichen Zellen zu produzieren, und
(iii) optional Züchten der genannten Population von menschlichen heptaoblast-ähnlichen Zellen auf einem Träger, der mit menschlichem Laminin beschichtet ist, in einem hepatischen Maturationsmedium, um eine Population von hepatozyt-ähnlichen Zellen, bevorzugt menschlichen fötalen hepatozyt-ähnlichen Zellen, zu produzieren,
und wobei das genannte Verfahren die Überexpression des Homeobox-Transgens *HEX* in der genannten Zellpopulation nicht umfasst.

4. Verfahren gemäß Anspruch 2 oder 3, wobei das hepatische Induktionsmedium ein chemisch definiertes Medium ist, umfassend morphogenetiches Knochenprotein 4 (BMP4) und einen Familienwachstums-Faktor (FGF 10 oder FGF2).

5. Verfahren gemäß Anspruch 2 oder 3, wobei das hepatische Maturationsmedium ein chemisch definiertes Medium ist, umfassend einen hepatischen Wachstumsfaktor (HGF) und Oncostatin M (OSM).

6. Verfahren gemäß Anspruch 1 oder irgendeinem der Ansprüche 3 bis 5, wobei das Entoderminduktions-Medium ein chemisch definiertes Medium ist, umfassend wenigstens Activin A und optional WNT3A.

7. Verfahren gemäß irgendeinem der Ansprüche 1 bis 6, wobei das hepatische Induktionsmedium und/oder das Entoderminduktions-Medium weiterhin einen ROCK-Inhibitor und/oder CHIR99021 umfasst.

8. Verfahren gemäß irgendeinem der Ansprüche 1 bis 7, wobei das menschliche Lamin (LN) aus der Gruppe ausgewählt ist, bestehend aus Lamin-111 (LN-111), Laminin-211 (LN-211), Laminin-332 (LN-332), Laminin-411 (LN-411), Laminin-421 (LN-421), Laminin-511 (LN-511) und Laminin-521 (LN-521).

9. Verfahren gemäß Anspruch 8, wobei das menschliche Laminin (LN) aus der Gruppe ausgewählt ist, bestehend aus menschlichem rekombinanten Laminin-111 (LN-111) und menschlichem rekombinanten Laminin-521 (LN-521) und einer Mischung davon.

## Revendications

1. Procédé d'induction de la différenciation hépatique humaine comprenant les étapes consistant à :
(i) mettre en culture une population de cellules pluripotentes ou multipotentes humaines sur un support revêtu de laminine humaine dans un milieu d'induction d'endoderme pour produire une population de cellules d'endoderme définitif (DE) humain, et dans lequel ledit procédé ne comprend pas la surexpression du transgène de boîte homéotique *HEX* dans ladite population de cellules.

2. Procédé d'induction de la différenciation hépatique humaine comprenant les étapes consistant à :
(i) mettre en culture une population de cellules d'endoderme définitif humain (DE) sur un support revêtu de laminine humaine dans un milieu d'induction hépatique pour produire une population de cellules de type hépatoblaste humain, et
(ii) éventuellement, mettre en culture ladite population de cellules de type hépatoblaste humain sur un support revêtu de laminine humaine dans un milieu de maturation hépatique pour produire une population de cellules de type hépatocyte, de préférence, des cellules de type hépatocyte foetal humain,
et dans lequel ledit procédé ne comprend pas la surexpression du transgène de boîte homéotique *HEX* dans ladite population de cellules.

3. Procédé d'induction de la différenciation hépatique selon la revendication 1 comprenant, en outre, les étapes de :
(ii) mise en culture de ladite population de cellules DE humaines sur un support revêtu de laminine humaine dans un milieu d'induction hépatique pour produire une population de cellules de type hépatoblastes humains, et
(iii) éventuellement, mise en culture de ladite population de cellules de type hépatoblastes humains sur un support revêtu de laminine humaine dans un milieu de maturation hépatique pour produire une population de cellules de type hépatocyte, de préférence, des cellules de type hépatocyte foetal humain,
et dans lequel ledit procédé ne comprend pas la surexpression du transgène de boîte homéotique *HEX* dans ladite population de cellules.

4. Procédé selon la revendication 2 ou 3, dans lequel le milieu d'induction hépatique est un milieu chimiquement défini comprenant une protéine morphogénétique osseuse 4 (BMP4) et un facteur de croissance familial (FGF 10 ou FGF2) .

5. Procédé selon la revendication 2 ou 3, dans lequel le milieu de maturation hépatique est un milieu chimiquement défini comprenant un facteur de croissance hépatique (HGF) et de l'oncostatine M (OSM).

6. Procédé selon la revendication 1 ou l'une quelconque des revendications 3 à 5, dans lequel le milieu d'induction d'endoderme est un milieu chimiquement défini comprenant au moins de l'Activine A et éventuellement du WNT3A.

7. Procédé selon l'une quelconque des revendications 1 à 6, dans lequel le milieu d'induction hépatique et / ou le milieu d'induction d'endoderme comprend, en outre, un inhibiteur de ROCK et / ou CHIR99021.

8. Procédé selon l'une quelconque des revendications 1 à 7, dans lequel la laminine humaine (LN) est choisie dans le groupe consistant en laminine-111 (LN-111), laminine-211 (LN-211), laminine-332 (LN-332), laminine-411 (LN-411), laminine-421 (LN-421), laminine-511 (LN-511) et laminine-521 (LN-521) .

9. Procédé selon la revendication 8, dans lequel la laminine humaine (LN) est choisie dans le groupe consistant en laminine-111 recombinante humaine (LN-111) et laminine-521 recombinante humaine (LN-521) et un mélange de celles-ci .
